# EUROPEAN PATENT APPLICATION

(11) **EP 3 417 889 A1**
(43) Date of publication of application: **26.12.2018**
(21) Application number: 18186867.0
(22) Date of filing: 03.12.2010
(51) Int. Cl.: A61L 27/56, A61L 27/20, A61L 27/12, A61L 27/38, A61F 2/28, A61L 27/46

(54) **BIOACTIVE GRAFTS AND COMPOSITES**

(30) Priority: 13.12.2009 US 63675109; 13.12.2009 WO PCT/US2009/067799
(62) Divisional of application: 10836450.6
(71) Applicant: Govil, Amit Prakash, Irvine, CA 92618 (US)
(72) Inventor: GAMBOA, Christian, Ladera Ranch, CA 92618 (US); GOVIL, Amit Prakash, Irvine, CA 92618 (US)
(74) Representative: Williams Powell

(57) **Abstract**

Disclosed are various bioactive grafts and/or biocompatible materials and methods of making the same. In one embodiment, bone material is harvested from a donor. The harvested bone material is exposed to a lysing agent, the lysing agent configured to release growth factors and bioactive materials from cellular material of the harvested bone material. The harvested bone material is then rinsed with a rinsing agent. The pH of the harvested bone material is substantially neutralized. In another embodiment, an orthopaedic implant includes an antibacterial polysaccharide. The implant may also include an osteostimulative agent.

## Description

### CROSS-REFERENCE TO RELATED APPLICATIONS

This application is a continuation in part of U.S. Patent Application Serial No. 12/636,751 entitled "BIOACTIVE GRAFTS AND COMPOSITES," filed December 13, 2009, which is incorporated by reference herein in its entirety, and which claims the benefit of U.S. Provisional Application Serial No. 61/201,612 entitled "STIMULATIVE GROWTH AGENTS DERIVED FROM PHYSIOLOGICAL FLUIDS AND METHOD OF MAKING," filed December 13, 2008, which is incorporated by reference herein in its entirety, and the benefit of U.S. Provisional Application Serial No. 61/240,283 entitled "BIOACTIVE ALLOGRAFTS AND COMPOSITES," filed September 7, 2009, which is incorporated by reference herein in its entirety.

### BACKGROUND

Bone grafts, whether an allograft, autograft or xenograft can be employed in patients suffering from painful or otherwise abnormal conditions related to instabilities or abnormalities in the skeletal structure. As a non-limiting example, a patients suffering from a spinal instability or excess movement of one or more vertebrae may be treated with a spinal fusion procedure involving removal of a portion of an intervertebral disc located between two vertebrae. A bone graft or spinal implant or a combination of both can then be inserted into or around the area of removed intervertebral disc to facilitate the fusion of two adjacent vertebrae. Such bone grafts or spinal implants can comprise harvested bone fragments made of cortical, cancellous, corticocancellous or a combination of all three aforementioned types of bone material. Patients may also suffer from various degenerative conditions for which implantation of a bone graft can be chosen as a treatment.

### BRIEF DESCRIPTION OF THE DRAWINGS

Many aspects of the present disclosure can be better understood with reference to the following drawings. The components in the drawings are not necessarily to scale, emphasis instead being placed upon clearly illustrating the principles of the disclosure. Moreover, in the drawings, like reference numerals designate corresponding parts throughout the several views.
FIG. 1 is a flow diagram illustrating one embodiment in accordance with the present disclosure.
FIG. 2 is a flow diagram illustrating one embodiment in accordance with the present disclosure.
FIG. 3 is a flow diagram illustrating one embodiment in accordance with the present disclosure.
FIG. 4 is a flow diagram illustrating one embodiment in accordance with the present disclosure.
FIG. 5 is a flow diagram illustrating one embodiment in accordance with the present disclosure.
FIG. 6 is a flow diagram illustrating one embodiment in accordance with the present disclosure.
FIG. 7 is a flow diagram illustrating one embodiment in accordance with the present disclosure.
FIG. 8 is a flow diagram illustrating one embodiment in accordance with the present disclosure.
FIG. 9 is a flow diagram illustrating one embodiment in accordance with the present disclosure.
FIG. 10 is a flow diagram illustrating one embodiment in accordance with the present disclosure.
FIGS. 11 is a flow diagram illustrating one embodiment in accordance with the present disclosure.
FIGS. 12-13 are flow diagrams illustrating methods to produce various embodiments of chitosan/mineral putty in accordance with the present disclosure.
FIGS. 14-17 are flow diagrams illustrating methods to produce various embodiments of chitosan/bone putty in accordance with the present disclosure.
FIGS. 18-20 are flow diagrams illustrating methods to produce various embodiments of chitosan/mineral scaffold sponge in accordance with the present disclosure.
FIGS. 21-26 are flow diagrams illustrating methods to produce various embodiments of chitosan/bone scaffold sponge in accordance with the present disclosure.
FIG. 27 is a table illustrating examples of material properties in accordance with various embodiments of the present disclosure.
FIGS. 28-29 are graphs illustrating examples of scaffold expansion in accordance with various embodiments of the present disclosure.

### DETAILED DESCRIPTION

Various embodiments of the present disclosure relate to bioactive factors and/or biocompatible materials that stimulate tissue growth. As can be appreciated these bioactive factors can be derived from physiological solutions containing cells. Physiological solutions may exist as solutions naturally in the body or be derived from tissue when the cells are extracted. Any tissue containing cells may be a source of physiological fluid, such as, for example, mesodermal, endodermal, and ectodermal tissues. Examples of these tissues include bone marrow, blood, adipose, skin, muscle, vasculature, cartilage, ligament, tendon, fascia, pericardium, nerve, and hair. These tissues may also include organs such as the pancreas, heart, kidney, liver, intestine, stomach, and bone. The cells may be concentrated prior to processing as described by the current disclosure.

One embodiment of the present disclosure relates to osteoinductive implants made from cellular bone tissue as well as methods of making osteoinductive implants. Implants made from cellular bone tissue can include osteoinductive and/or osteoconductive materials to facilitate fusion and/or new bone growth in or around an area of implant insertion. Accordingly, in accordance with one embodiment, a portion of cancellous, corticocancellos and/or cortical bone or any combination thereof can be harvested from a donor. In one embodiment, the harvested material can be harvested in such a way as to retain as much bone marrow in the harvested sample as possible.

The harvested sample can be exposed to lysing conditions and/or a lysing agent to facilitate lysis of the cells therein to release growth factors and nutrients contained sample. In other words, the harvested sample can be exposed to a lysing agent that lyses the cells within the harvested sample sample. Once cellular components are lysed, they release growth factors and/or bioactive materials, such as cytokines and nutrients, to stimulate growth, differentiation, and repair. These growth agents can be cytokines such as proteins, hormones, or glycoproteins including members of the TGF-β family (including bone morphogenetic proteins), interleukins, interferons, lymphokines, chemokines, platelet derived growth factors, VEGF, and other stimulative agents that promote growth, repair or regenerate tissues.

In other embodiments, cells from other tissues can be lysed to release growth agents that can be binded to the harvested sample and further processed as an implant. Lysing conditions may be mechanical in nature such as thermolysis, microfluidics, ultrasonics, electric shock, milling, beadbeating, homogenization, french press, impingement, excessive shear, pressure, vacuum forces, and combinations thereof. Excessive shear may be induced by aggressive pipetting through a small aperture, centrifuging at excessive revolutions per minute resulting in high gravity forces. Rapid changes in temperature, pressure, or flow may also be used to lyse cellular components. Lysing conditions can include thermolysis techniques that may involve freezing, freeze-thaw cycles, and heating to disrupt cell walls. Lysing conditions can also include microfluidic techniques that may involve osmotic shock techniques of cytolysis or crenation.

Lysing conditions can also include the imposition of ultrasonic techniques, including, but not limited to, sonication, sonoporation, sonochemistry, sonoluminescence, and sonic cavitation. Lysing conditions can also include electric shock techniques such as electroporation and exposure to high voltage and/or amperage sources. Lysing conditions can further include milling or beat beating techniques that physically collide or grind cells in order to break the cell membranes, releasing the stimulative agents contained within.

Lysing can also be accomplished by exposing cells of the harvested sample to a lysing agent, which can facilitate release of stimulative growth agents include lysis due to pH imbalance, exposure to detergents, enzymes, viruses, solvents, surfactants, hemolysins, and combinations thereof. Chemical induced lysis of the cells by pH imbalance may involve exposure of cells of the harvested sample to a lysing agent in order to disrupt the cell walls and release soluble growth agents. In some embodiments, a lysing agent can include one or more acids and/or bases.

After exposure to the lysing agent, the harvested sample may be exposed to buffers or other solutions to substantially neutralize the pH of the mixture of the growth factors and the lysing agent. In some embodiments, it may be desired that the pH be acidic (*e.g.,* pH below 7) or basic (*e.g*., pH above 7) to retain solubility of particular growth factors or bioactive agents. For example, bone morphogenetic proteins (particularly BMP-2, BMP-4, BMP-6, BMP-7, BMP-9, BMP-14, and other bone morphogenetic proteins 1-30) are more soluble at acid pH values under 7 than neutral or basic pH.

In other embodiments, a lysing agent can include a volatile acid or base, such as acetic acid or ammonia, and the cellular material, after exposure to the lysing agent, may be neutralized or partially neutralized by drying techniques such as evaporation, vacuum drying, lyophilization, freeze drying, sublimation, precipitation, and similar processes as can be appreciated. In yet other embodiments, a lysing agent can include detergents that can disrupt cell walls and remove any lipid barriers that may surround the cell. Enzymes, viruses, solvents, surfactants, and hemolysins can also help cleave or remove outer cell membranes releasing the bioactive growth agents contained within.

The use of these lysing agents and/or exposure of the harvested sample to lysing conditions may be followed by neutralization, as noted above, and/or another secondary process to remove any undesired remnants. The growth agents, nutrients, *etc.,* released by the lysing process may be added to a carrier such as a synthetic scaffold, biologic scaffold, and autologous, allogeneic, and xenograft tissue. In yet other embodiments, a harvested sample acting as a carrier can be exposed to lysing conditions and/or a lysing agent, and growth factors released by the lysing process can be binded to at least a portion of the sample. In other words, the growth agents released by lysing of cellular material may be used immediately for autologous use. In other embodiments, the released growth agents may be stored for allogenic use. Storage techniques can include freezing or lyophilization to preserve bioactivity. The growth factors and nutrients may also be frozen or lyophilized on the chosen carrier to allow for complete binding of the stimulative agent to the carrier and to allow for immediate use by the surgeon. Lyophilization also allows for greater room temperature shelf life and an opportunity for concentration into a smaller volume.

Another embodiment of the present disclosure relates to obtaining a specific set of growth factors and nutrients from a physiological solution containing cells. In this embodiment, cells are lysed as described above and the lysate solution is subjected to materials with a charged surface, including, but not limited to, chromatography resins, ceramics, mineralized tissues, demineralized tissues, soft tissues, and other materials with an electric charge. The charged surface attracts certain stimulative growth agents and molecules removing them from the lysate solution. The remaining growth agents can then be used to regenerate or repair the desired tissue type. Similar to the previous embodiment, the growth agent solution can be further concentrated and frozen or lyophilized in order to extend shelf life.

Another embodiment of the disclosure includes selectively rinsing, lysing, or removal of certain cellular components while retaining other cellular components. Selective lysing or removal can be accomplished physically by methods described above. As can be appreciated, certain cells can be resistant to various lysing mechanisms. As a non-limiting exmaple, mesenchymal stem cells (MSC) are resistant to cytolysis and osmotic lysis due to their resistant cell walls and ineffective cells volumes. Accordingly, to accomplish selective lysing, osmotic lysis can be used to lyse red and white blood cells from blood or bone marrow. Once the non-resistant cells are lysed, the resulting solution is an enriched MSC population. The solution can then be further concentrated via centrifugation, florescence-activated cell sorting (FACS), filtration, magnetic bead selection and depletion, and/or gravity sedimentation. For allogeneic transplantation, FACS and magnetic bead separation and depletion can be useful in removing any remaining cells that would cause an immune response from the implant patient. Once implanted, cells can function in a homologous manner and differentiate in the desired phenotype.

Another embodiment of the disclosure includes a combination of previous two embodiments. A physiological solution may be enriched by selective lysis and further concentrated by centrifugation, FACS, magnetic bead selection and depletion, and/or gravity sedimentation. The enriched physiological solution is added to a physiological solution that has been lysed in the methods described previously in order to help induce differentiation of the cells into the desired phenotype. These cells can then function in the desired manner to regenerate and repair tissues.

In another embodiment, cancellous bone may be exposed to a weak lysing agent (such as less than 1M acetic acid) that only partially lyses the cell population present. In this embodiment, the partial lysis releases growth factors and binds them to the bone while other cells, such as mesenchymal stem cells and progenitor cells, may still remain viable and attached to the bone.

In another embodiment, cancellous bone may be exposed a weak lysing agent (such as water) and then subjected to mechanical lysing conditions previously stated (such as thermolysis, high/low pressure, sonication, centrifugation, *etc*.). Once the cells have lysed, the bone, cell fragments, and debris are removed from the solution containing the growth factors. The solution may then become positively charged by the addition of an acid or another proton donor fluid. The growth factors in the solution may then be further concentrated using techniques described, frozen, or lyophilized into a soluble powder. The soluble powder could be reconstituted with a fluid prior adding it to an implant during surgery or added in the dry powder form to an implant prior to implantation.

In another embodiment, an osteoinductive growth factor can be formed from physiological fluids containing cells. These cells are lysed as previously described and may be loaded onto allograft bone from the same tissue donor as the cells. The stimulative growth agents may be loaded onto the bone prior to lyophilization or freezing. The bone may be mineralized or demineralized prior to loading of the stimulative growth agents to allow for more complete bonding of the stimulative growth agents. The bone may also be morselized prior to or after loading with stimulative growth agents allowing it to be used in a flowable composition.

In another embodiment, a physiological fluid containing cells, such as synovial fluid, may be harvested from a live donor, cadaveric donor, or autologously. The fluid may be subjected to mechanical or chemical lysing conditions described in order to solubilize growth factors. Once the growth factors are released from the cells, the solid materials (such as cells fragments, debris, or platelets) may be removed by processes described such as filtration, centrifugation, or gravity sedimentation. Once the solid materials are removed, the solution may be then become positively charged by the addition of an acid or another proton donor fluid. The growth factors in the solution may then be further concentrated using techniques described, frozen, or lyophilized into a soluble powder. The soluble powder could be reconstituted with a fluid prior adding it to an implant during surgery or added in the dry powder form to an implant prior to implantation. Alternatively, cartilage with or without synovial fluid can be prepared in a similar fashion for the repair and regeneration of cartilage or spinal discs. In addition, other tissues such as muscle, adipose, nerve, dermis, cardiac tissue, vascular tissue, nucleus pulposus tissue, annulus fibrosus tissue, or other solid tissues can be prepared in this fashion to be used to help repair or regenerate tissues.

Stimulative growth agents can be derived from various cellular solutions. These solutions may comprise cultured and/or uncultured cells, and can be autologous, allogeneic, or xenogeneic in origin. If the cells are allogeneic or xenogeneic in origin, at least partial lysing or immune cells depletion by methods previously described can be performed so that the stimulative growth agents do not elicit an immune response in the patient. Alternatively, immune response agents, such as CD45+ cells and other leukocytes, may be removed prior to use to reduce or eliminate immune response. These immune response agents may be removed by the selective lysing as previously described in this disclosure.

Various embodiments of the present disclosure relate to compositions and/or methods for providing an anti-microbial polysaccharide scaffold that may be combined with an osteostimulative agent such as bioactive growth factors and different types of cells to stimulate tissue growth, cell adhesion, cell proliferation, and enhanced wound healing. Chitosan is a polysaccharide found in marine crustacean shells and the cell walls of bacteria and fungi. Chitosan is a non-toxic biocompatible material that can support tissue growth. With the combination of biocompatibility, antibacterial activity, versatility in processing, and ability to bind cells and growth factors, chitosan is a distinguished biomaterial to support in tissue growth. Materials may be customized for use within the applications such as, but not limited to; bone void filler, musculoskeletal defect, non-structural bone defect, structural bone defect, intervertebral space, intertranseverse space, implant coating, hemostatic agent, wound covering, osteoncology, and treatment of infected site. The scaffold may also include minerals.

In one embodiment, a biocompatible shape memory osteoconductive and/or osteoinductive anti-microbial compressible implant scaffold may be used in tissue engineering. For example, the present disclosure provides an orthopedic structure comprising a chitosan solution and a non-toxic mineral mixture resulting in a compressible solid porous substrate.

The scaffold may comprise chitosan with a weight percentage in the range of about 5% to about 80%, in the range of about 10% to about 70%, and/or in the range of about 15% to about 60%. In some embodiments, the chitosan concentration is greater than about 5%, greater than about 30%, or more. In other embodiments, the chitosan concentration is less that about 10% or less than about 2.5%.

In accordance with various implementations of the present disclosure, the chitosan molecular weight may be in a range of between about 1 kDa and about 750 kDal, in a range of between about 10 kDal and about 650 kDa, and/or in a range of between about 50 kDa and about 550 kDa.

The chitosan used may be deacetylated chitosan. According to one implementation, the degree of deacetylation may range from, but is not limited to, about 50% to about 99% deacetylation. Generally, the lower the percentage/degree of deacetylation, the more rapid the degradation takes place when implanted. The deacetylation percentages may also be tailored to specific tensional and compressive properties. The lower the deacetylation the lower the tensile strength of the scaffold.

In accordance with various implementations, the deacetylation percentage of the chitosan can be in a range from about 50% to about 66.6% in order to produce more rapid degradation profile and in turn have a lower density affecting porosity. In other implementations, the deacetylation percentage of the chitosan can be in a medium range from about 66.6% to about 83.2% in order to produce a medium degradation profile and in turn have a medium density affecting porosity. In accordance with yet other implementations, the deacetylation percentage of the chitosan can be in a medium range from about 83.2% to about 99% in order to produce a longer degradation profile and in turn have a higher density affecting porosity.

The chitosan material may be compounded with an additional protein or amino acid to improve protein and cell binding. Examples of proteins, enzymes, structural proteins, cell signaling or ligand binding proteins, or amino acids include, but are not limited to, collagen, glutamic acid, and lysine. The chitosan may be medical grade or may be of equivalent quality containing low level of toxic contaminants such as heavy metals, endotoxins and other potentially toxic residuals or contaminants.

In accordance with various embodiments of the present disclosure, the chitosan solution can be prepared by dissolution in low pH fluids, such as acids. Low pH fluids include, but are not limited to, acetic, hydrochloric, phosphoric, sulfuric, nitric, glycolic, carboxylic, or amino acids. The amount of acid used may be between about 0.1% to about 50%, and/or may be between about 0.1% and about 25%. In some embodiments, the pH can range from slightly acidic to neutral or partially neutral. Neutralization can be obtained by using base substances such as, but not limited to, sodium hydroxide, ammonia hydroxide, potassium hydroxide, barium hydroxide, caesium hydroxide, strontium hydroxide, calcium hydroxide, lithium hydroxide, rubidium hydroxide, butyl lithium, lithium diisoprpylmadie, lithium diethylamide, sodium amide, sodium hydride, and lithium bis(trimethylsily)amide. Neutralization may also be obtained by using basic amino acids including lysine, histidine, methyllysine, arginine, argininosuccinic acid, L-arginine L-pyroglutamate, and ornithine. Different techniques to achieve neutralization may be used such as evaporation, vacuum drying, lyophilization, freeze drying, sublimation, precipitation, and similar process as can be appreciated. The resulting solution results in a suspension or gel comprising chitosan with a liquid medium being at least partially comprised of water. The suspension or gel may also include tissue and/or mineral particles. Tissue may include allograft, autograft, or xenograft.

The resultant chitosan/mineral suspension may then be shaped to desired forms such as porous solids or semisolids through techiniques such as injection molding, vacuum molding, injection compression molding, rotational molding, electrospinning, 3D printing, casting, and phase separation. The shapes may be orthopedic shapes such as, for example, dowels, tubes, pins, screws, plates , wedges anchors, strips, bands, hooks, clamps, washers, wires, fibers, rings, sheets, spheres, and cubes.

In accordance with another aspect of the disclosure, the chitosan scaffold may have a matrix porosity ranging from about 1µm to about 5mm. The matrix scaffold may also have a different surface porosity compared to its internal porosity. The surface porosity may have ranges from about 1 µm to about 1 mm, while the internal porosity may range from about 10 µm to about 5 mm. Overall pore size can be dependent on concentrations of chitosan, lower concentrations will result in larger pore size while higher concentration will result in smaller pore size. Pores size may also be designed to align vertically, longitudinally, horizontally, or a combination thereof depending on the process used during preparation or the intended site of implantation. Size and direction of the pores and channels may be designed and controlled through control rate freezing, and directional freezing. Variables such as freezing rate, freezing temperature, and specified area of freezing can be changed to adjust pore/channel size and direction due to the functions of the temperature gradient. An implant can be frozen at a ramp down rate of -0.1°C to -15°C every 1 minute to 20 minutes, creating uniform crystal formation. After freeze drying, the crystals evaporate leaving pores within the implant. For example, a slow ramp down rate of -10°C every 10 minutes will result in larger pore size, while a fast ramp down rate of -10°C every 1 minute results in smaller pore size. Channels instead of pores can be formed by decreasing the ramp down rate even further to -5°C every 15 minutes. Pore and/or channel directionality can designed by applying the freezing source during freeze drying to a specified area of the implant. For example, if the freezing source is applied to a specified area (*e.g*., a specific surface) of the implant, the pore or channel direction will be perpendicular to the freezing source. A combination of applied freezing sources can result in multidirectional pore or channel structure. If the freezing source is not placed in any specified area, then the pore or channel direction can be anisotropic.

In accordance with another aspect of the present disclosure, the implant may have shape memory once hydrated with liquid. A dehydrated or hydrated sponge may be compressed circumferentially, unilaterally, or in multiple directions up to about 10 times its original size but when hydrated goes back to its original shape. The scaffold can be compressed into various shapes such as, but not limited to, tubes, pins, cubes, strips, and sheets. Compression may occur externally directed towards the scaffold or internally directed outward from the scaffold.

In some embodiments, the biocompatible implant may include minerals such as calcium salts (*e.g*., calcium phosphate), silicate, carbonate, sulfate, halide, oxide sulfide phosphate, metals or semimetals including gold silver copper, alloys, and/or a combination thereof. In accordance with one aspect of the present embodiment, calcium phosphate may be selected from hydroxyapatite (HA), silicate hydroxyapatite (HA), tri-calcium phosphate (TCP), pure/substituted beta tri-calcium phosphate (β-TCP), alpha tri-calcium phosphate (α-TCP), octalcalcium phosphate (OCP), tetralcalcium phosphate (TTCP), dicalcium phosphate dehydrate (DCPD), and/or a combination thereof. Mineral particle sizes may range from a powder of about 1 nm to about 1 mm. The mineral content can also be added in a granule size ranging from about 50 µm up to about 5 millimeters. The implant may include granules larger than 100 µm to increase compression resistance and cell/protein binding. The calcium salt concentration may be greater than about 10%, greater than about 30%, or greater than about 40%.

The scaffold may comprise a mineral in a range of about 5% to about 75%, in a range of about 8% to about 72%, and/or in a range of about 10% to about 70%. In accordance with yet another aspect of the embodiment, the scaffold can be comprised of a mineralized, demineralized, or partially demineralized bone in a range of about 5% to about 75%, in a range of about 8% to about 72%, and/or in a range of about 10% to about 70%.

In accordance with yet another aspect of the disclosure, the implant contains antimicrobial and/or antibacterial properties which are dependent on the amount of chitosan and pH levels that are used in the formulation. The chitosan concentration along with the pH can provide antimicrobial activity against but not limited to the following organisms; staphlyococcus aureus (MRSA), enterococcus faecalis (VRE), acinetobacter baumanii, escherichia coli, klebsiella pneumoniae, streptococcus pyogenes, staphylococcus epidermidis, alomonella choleraesuis, pseudomonas aeruginos, enterococcus faecalis, serratia marcescens, stenotrphomonas maltophilia, streptococcus mutans, clostrium difficle, streptococcus pneumoniae, shigella species, enterobacter aerogenes, proteus mirabilis, proteus vulgaris, citrobacter freundii, enterobacter cloacae, moraxella catarrhalis, micrococcus luteus, and vibrio cholera. The material also increases in stiffness after an increase in pH. In some embodiments, the chitosan solution can range from about 5 mg/mL to about 200 mg/mL. The pH level may be less than 8 and/or less than 7.

In accordance with various embodiments, the scaffold tensile, torsional,shear, and compressive properties can be strengthened by crosslinking using methods such as, dehydrothermal, chemical, physical, or photometric crosslinking. Dehydrothermal crosslinking may involve exposing the scaffold to elevated temperatures with or without the use of negative pressure. Chemical crosslinking may include treatment with nitrous acid, malondiadehyde, psoralens, aldehydes, formaldehydes, gluteraldehydes, acetalaldehyde, propionaldehyde, butyraldehyde, bensaldehyde, cinnamaldehyde, and/or tolualdehyde. Photometric crosslinking may use energy and/or light sources that may include ultraviolet, plasma, or other energy sources.

In one embodiment, a biocompatible shape memory osteoconductive and/or osteoinductive anti-microbial compressible implant scaffold may be used in tissue engineering. An orthopedic structure comprising a chitosan solution and a bone mixture results in a compressible solid porous substrate. The bone may be selected from allograft bone, xenograft bone, autograft bone, or a combination thereof. The bone may be fine or coarse ground powder or porous granules. For example, the granules may be greater than about 100 µm to increase compression resistance or cell/protein binding. The powder can be homogeneously or heterogeneously integrated throughout the scaffold depending on the application. The bone may also be mineralized, demineralized, partially demineralized, solubilized, or gelatinized.

In an alternate embodiment, a biocompatible shape memory osteoconductive and/or osteoinductive anti-microbial implant scaffold maybe used in tissue engineering. An orthopedic structure comprising a chitosan solution and a mineral/bone mixture results in a compression resistant substantially solid yet porous substrate. The scaffold may include larger particle sizes of mineral and bone (*e.g*., greater than about 100 µm) to aid in compression resistance.

In other embodiments, a biocompatible osteoconductive and/or osteoinductive anti-microbial implant scaffold may be used use in tissue engineering. An orthopedic structure comprising a chitosan solution and a mineral/bone mixture results in a load bearing implant. For example, the load bearing or compression resistance may be enhanced by crosslinking, increased density, and/or larger particle sizes.

In accordance with one implementation, the scaffold may consist of demineralized or partially demineralized bone and chitosan. The chitosan may be in a range of about 0.1% to about 20% and/or in a range of about 0.5% to about 15%. In accordance with a second implementation, the scaffold may consist of calcium salt and chitosan. The chitosan may be in a range of about 0.1% to about 20% and/or in a range of about 0.5% to about 15%.

In various embodiments, a biocompatible osteoconductive and/or osteoinductive anti-microbial implant scaffold may be used use in tissue engineering. An orthopedic structure comprising a chitosan solution includes one or more substances including growth factors, growth factor stimulative agents, vitamins, and/or biologically active molecules. Calcium salts (*e.g*., calcium phosphate) and/or tissue (*e.g*., bone) may also be included as an osteostimulative agent.

Growth factors may be bound to the scaffold. Growth factors include, but are not limited to, bone morphogenetic protein (BMP), transforming growth factor β (TGF-β), growth differentiation factor (GDF), cartilage derived morphogenetic protein (CDMP), interlukins, interferon, lymphokines, chemokines, platelet derived growth factors (PDGF), VEGF, β-fibroblast growth factor (β-FGF), fibroblast growth factors (FGF), and other stimulative agents that promote growth, repair or regenerate tissue. Bone morphogenetic protein may be selected from BMP-2, BMP-3, BMP-4, BMP-5, BMP-6, BMP-7, BMP-8, BMP-9, BMP-10, BMP-11, BMP-12, BMP-13, BMP-14, BMP-15, and BMP-16. The bone morphogenetic protein may also be recombinant human bone morphogenetic protein. Growth factors may also be angiogenic or mitogenic growth factors

In another embodiment, a biocompatible osteoconductive and/or osteoinductive anti-microbial implant scaffold may be used in tissue engineering. An orthopedic structure comprising a chitosan solution and a mineral/bone mixture includes seeded cells. The cells can comprise of mesechymal stems cell (MSC), adipocytes, chondrocytes, osteocytes, fibroblasts, osteoblasts, preosteoblasts, osteprogenitor cells, and combinations thereof.

In various embodiments, a biocompatible osteoconductive and/or osteoinductive anti-microbial malleable implant scaffold may be used in tissue engineering. An orthopedic structure comprising a chitosan solution and a mineral/bone mixture has a putty-like consistency. The material may be molded to meet different situations. Formulation parameters may be adjusted to have different viscosities and adhesion characteristics based on the application.

In alternative embodiments, a biocompatible osteoconductive and/or osteoinductive anti-microbial flowable implant scaffold may be used in tissue engineering. An orthopedic structure comprising a chitosan solution and a mineral/bone mixture has a flowable consistency. The material may be tailored to meet different situations. Viscosity parameters may be formulated to have less viscous properties in applications such as pastes, injectable gels and sprays. The paste and gels can be applied into the body in the desired shape, to aid in the efficacy of the application. A less viscous formulation such as putty or a very viscous injectable/flowable fluid can be applied in places such as bone voids, bioinert implants, cannulated screws, around screws, or other orthopaedic applications.

In various other embodiments, a biocompatible osteoconductive and/or osteoinductive anti-microbial coating implant scaffold may be used in tissue engineering. An orthopedic structure comprising a chitosan solution and a mineral and/or bone mixture has a low viscosity consistency for coating purposes. The coating may be applied to bioinert materials such as, but not limited to, peek, stainless steel, titanium, radel, and silicone structures. For example, a coating can be applied to (e.g., sprayed on) bioinert implants such as, but not limited to, cages, screws, screw heads, pins, rods, wires, dowels, connectors, hip stems, acetabular cups, and plates. A coating may also be applied to (e.g., sprayed on) bioactive implants such as, but not limited to, minerals, autograft, allograft, xenograft, demineralized bone, partially demineralized bone,mineralized bone, and collagen.

The systems and methods described herein can be employed in surgical environments where the implantation of stimulative growth agents in a patient is desired. Although the present disclosure describes the methods and systems for producing stimulative growth agents, particularly ones derived from physiological fluids containing cells or cellular tissues, it is understood that the methods and systems can be applied for a wide variety of medical applications including ones directed at regeneration or repair of bone, cartilage, muscle, tendon, ligament, vasculature, fat, annulus fibrosus, nucleus pulposus, skin, hair, blood, lymph nodes, fascia, neural, cardiac, pancreatic, hepatic, ocular, dental, digestive, respiratory, reproductive, and other soft tissue applications, such as in regenerative medicine and tissue engineering.

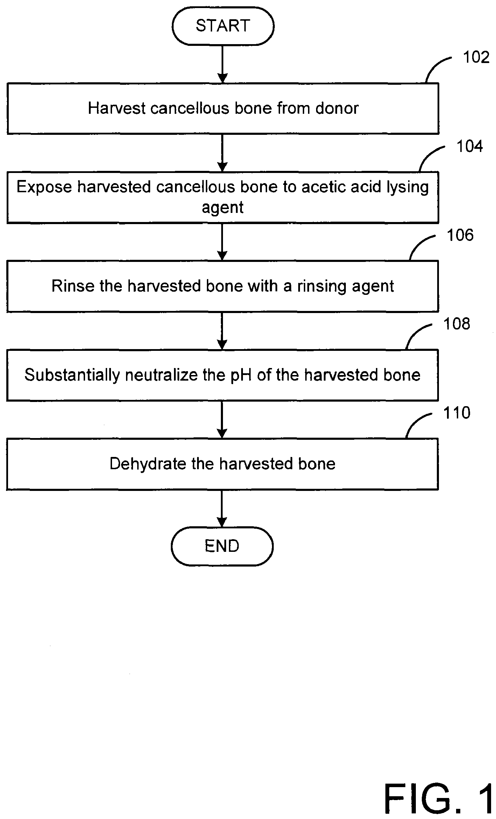

Reference is now made to FIG. 1, which depicts a method in accordance with one embodiment of the disclosure. In the embodiment illustrated in FIG. 1, an implant that can be suitable for bone applications is shown. In the embodiment of FIG. 1, cancellous bone is recovered from a cadaver, live donor, or harvested autologously from a patient in box 102. The harvested cancellous bone can be ground or cut to a desired shape and configuration as can be appreciated. Care may be taken to retain some cellular material, bone marrow, and/or blood within the bone during harvest and cutting operations. In prior art implants, bone marrow and/or blood within the bone can be systematically removed and/or cleaned from the harvested bone sample. In an embodiment of the disclosure, cancellous bone may have cortical bone portions such as in the iliac crest, vertebral bodies, chondyles, etc. Accordingly, in some embodiments, depending on the needs of a particular application, the cancellous bone may have cortical portions removed prior to further processing.

The cancellous bone is then exposed to acetic acid in box 104, which acts as a lysing agent as described above. In one embodiment, the acetic acid concentration can be greater than 1%, in a molarity range of 0.2M - 17M. The acetic acid lysing agent is employed to lyse cells remaining in the porous bone structure and on bone surface of the cancellous bone. The lysing of the cells releases and solubilizes growth factors and bioactive materials contained in the cellular material. The acetic acid lysing agent also allows the solubilized bioactives to bind to the bone. The bone may be further rinsed and cleaned by a rinsing agent in box 106 after exposure to the acetic lysing agent and after growth factors and/or bioactive materials bind to the bone. Rinsing can be conducted in order to remove excess acetic acid, cell fragments, lipids, and/or debris. Additionally, pH of the harvested bone may be substantially neutralized in box 108. In some embodiments, the pH of the harvested bone can be neutralized by the rinsing agent and rinsing step in box 106. In other embodiments, pH neutralization may not be required. Further pH neutralization of the harvested bone may be accomplished by dehydrating in box 110 by evaporation, vacuum drying, or lyophilization to reduce the acetic acid lysing agent to a residue and bring the implant to a more neutral pH.

Rinsing solutions can be water, saline (NaCl, PBS, *etc*.), peroxides, alcohol (isopropyl, ethanol, *etc*.), crystalloids, sterilizing fluids (antibiotics such as gentamicin, vancomycin, bacitracin, polymixin, amphotericin, ampicillin, amikacin, teicoplanin, *etc*.), preserving fluids (DMEM, DMSO, mannitol, sucrose, glucose, *etc*.), storage agents, and/or other fluids used in processing of allografts. The resulting product yields a cancellous bone implant with increased bioacvitity. In some embodiments, ground particulate filler implants as well as structural cancellous bone implants with increased bioactivity may be formed.

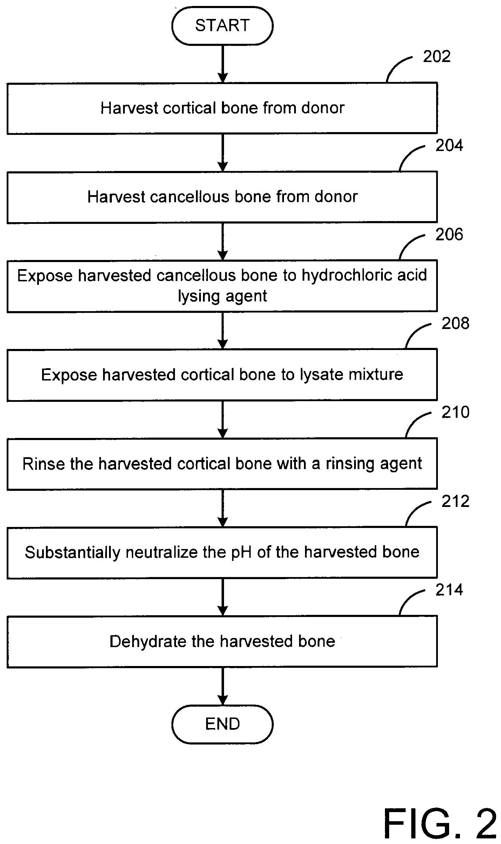

Reference is now made to FIG. 2, which depicts an alternative embodiment of the disclosure. The depicted flow diagram illustrates a method of forming an implant made from harvested cortical bone with bioactives and growth factors from harvested cancellous bone binded to the cortical bone material. In the depicted embodiment, cortical bone is harvested in box 202 from a cadaver, live donor, and/or harvested autologously from a patient. Cancellous bone is also harvested in box 204 from the same donor. The harvested cortical bone may be ground or cut to a desired shape and configuration depending on a particular application desired. The cortical bone may be cleaned and demineralized (*e.g*., with hydrochloric acid washes and/or treatment with citric acid) to remove its mineral content. The harvested cancellous bone may also be ground or cut to a particular shape or configuration depending on the application desired. Care may be taken to retain as much bone marrow and blood within the cancellous bone during harvest and cutting operations. Cancellous bone may have cortical bone portions such as in the iliac crest, vertebral bodies, chondyles, *etc*.

Accordingly, in some embodiments, depending on the application of an implant, the cancellous bone may have cortical portions removed prior to further processing. The cancellous bone is then exposed to hydrochloric acid (*e.g*., 0.1M - 16M) as a lysing agent in box 206 to lyse cells remaining in the porous bone structure and on the bone surface. The lysing of the cells releases and/or solubilizes growth factors and bioactive materials contained in the cellular material. In contrast to the embodiment disclosed above in FIG. 1, hydrochloric acid can be employed as a lysing agent that restricts the solubilized growth factors and bioactives from binding to the cancellous bone, but they are present in the hydrochoric acid and lysate mixture. The solubilized growth factors and bioactives in the lysate mixture are then added to the cortical bone that is harvested from the same donor in box 208.

The growth factors and bioactives in the hydrochloric acid mixture readily bind to the mineralized and/or demineralized cortical bone (*e.g.,* 1 minute - 50 hour binding time). The cortical bone may be further rinsed and cleaned in box 210 after the binding to remove excess hydrochloric acid, cell fragments, lipids, and/or debris. Rinsing solutions can be water, saline, peroxides, alcohol, crystalloids, sterilizing fluids, preserving fluids, storage agents, or other fluids used in processing of allografts. The cortical bone can then undergo pH neutralization in box 212, which can be accomplished by dehydration in box 214 as is noted above in some embodiments. pH neutralization can also be accomplished by other chemical agents or physical processes as can be appreciated. Accordingly, ground particulate filler implants as well as structural cortical bone implants with increased bioactivity may be made in this manner.

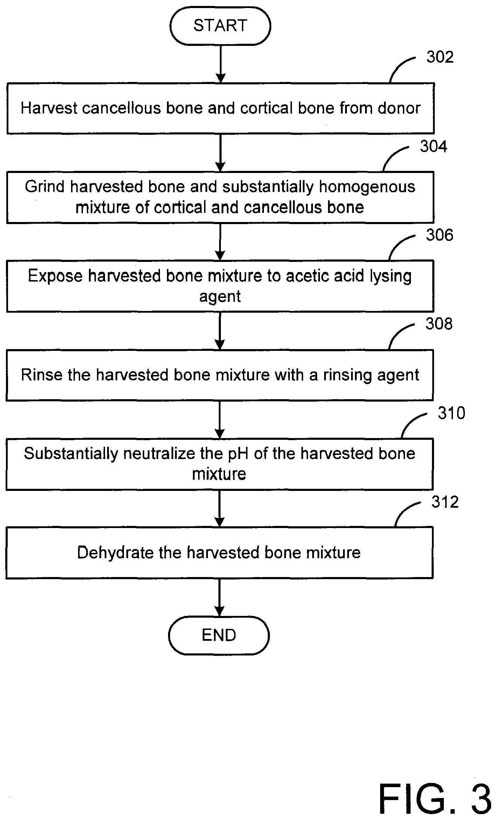

Reference is now made to FIG. 3, which depicts an alternative embodiment of the disclosure. Cortical bone and cancellous bone are harvested and/or recovered from a cadaver, live donor, or harvested autologously from a patient in box 302. If required by a particular implant application, cancellous and/or cortical bone may be ground or cut to a desired shape and configuration. Care is taken to retain as much cellular material, bone marrow, and/or blood within the bone during harvest and cutting operations. In the embodiment of FIG. 3, the harvested cancellous bone and harvested cortical bone are ground and then mixed to create a substantially homogenous mixture in box 304. Cortical bone may be demineralized using techniques of hydrochloric acid washes that are noted above prior to mixing with the cancellous bone if desired.

The cancellous and cortical bone mixture may further be homogenized by mixing with another fluid (such as water) so that the growth factors may be more homogenously distributed throughout the mixture. The solution containing bone is then exposed to acetic acid (*e.g*., 0.1M - 17M concentrations) as a lysing agent in box 306 to lyse cells remaining in porous bone structure and on bone surface. The lysing of the cells releases and solubilizes growth factors and bioactive materials contained in the cellular material. Acetic acid also allows the solubilized bioactives to bind to the cortical and cancellous bone mixture. Further acid washes may be desired to further demineralized the bone, reduce its modulus, and/or make it more spongy. Any type of acid including acetic, hydrochloric, citric, phosphoric, *etc.,* may be used to further demineralized the bone.

The bone may be further rinsed and cleaned in box 308 after the binding to remove excess acid, cell fragments, lipids, and/or debris. In some embodiments, the bone may be dehydrated by evaporation, vacuum drying, or lyophilization to remove any residual acetic acid and neutralize the pH of the cortical and cancellous bone mixture in boxes 310 and 312. Rinsing solutions can include water, saline, peroxides, alcohol, crystalloids, sterilizing fluids, preserving fluids, storage agents, or other fluids used in processing of allografts. Accordingly, ground particulate filler implants as well as structural corticocancellous bone implants with increased bioactivity may be made in this manner.

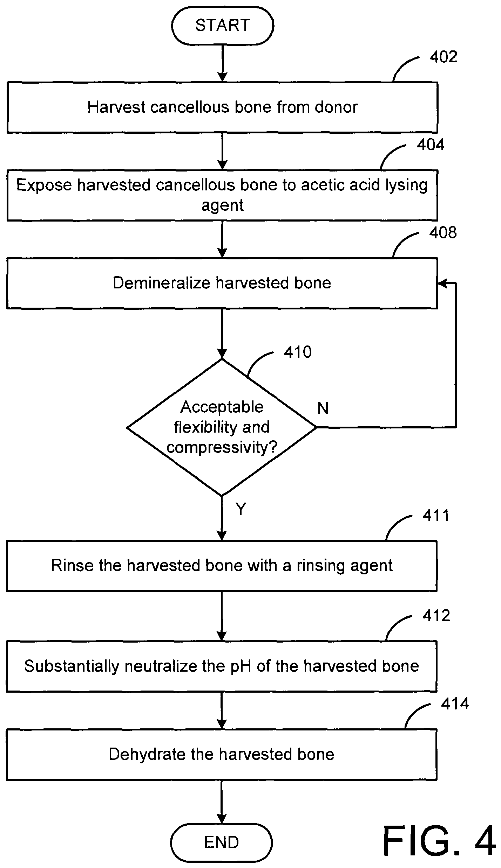

Reference is now made to FIG. 4, which depicts an alternative embodiment of the disclosure. Cancellous bone is recovered from a cadaver, live donor, or harvested autologously from a patient in box 402. If required by a particular implant application, the harvested cancellous bone may be ground or cut to a desired shape and configuration. Care may be taken to retain as much cellular material, bone marrow, and/or blood within the bone during harvest and cutting operations. Cancellous bone may have cortical bone portions such as in the iliac crest, vertebral bodies, chondyles, *etc*. Accordingly, cortical portions of cancellous bone may be removed from the cancellous bone. The cancellous bone can then be exposed to acetic acid (*e.g*., 0.1 M - 17M) as a lysing agent in box 404 to lyse cells remaining in porous bone structure and on bone surface. The lysing of the cells releases and solubilizes growth factors and bioactive materials contained in the cellular material. Acetic acid also allows the solubilized bioactives to bind to the bone. The cancellous bone may be further demineralized in box 408 using at least one demineralization wash using any acid, including, but not limited to, acetic, hydrochloric, citric, phosphoric, *etc*., to alter the mechanical properties of the bone and remove mineral content.

A compression test may be performed between demineralization washes to determine the whether the level of flexibility and compressivity of the bone is acceptable for a given application in box 410. If the bone is too rigid for a desired application, further demineralization washes may be performed. Once the desired flexibility is achieved, the bone may be further rinsed and cleaned in box 411 after the binding to remove excess acid, cell fragments, lipids, or depris. In some embodiments, the bone may be dehydrated by evaporation, vacuum drying, or lyophilization to residual any residual acetic acid and bring the implant to a more neutral pH in boxes 412 and 414. It should be appreciated that pH neutralization can be accomplished by chemical agents or physical processes other than by dehydration. Rinsing solutions can be water, saline, peroxides, alcohol, crystalloids, sterilizing fluids, preserving fluids, storage agents, *etc.,* or other fluids used in processing of allografts. Accordingly, ground particulate filler implants as well as structural but flexible/compressible cancellous bone implants with increased bioactivity may be made in this manner.

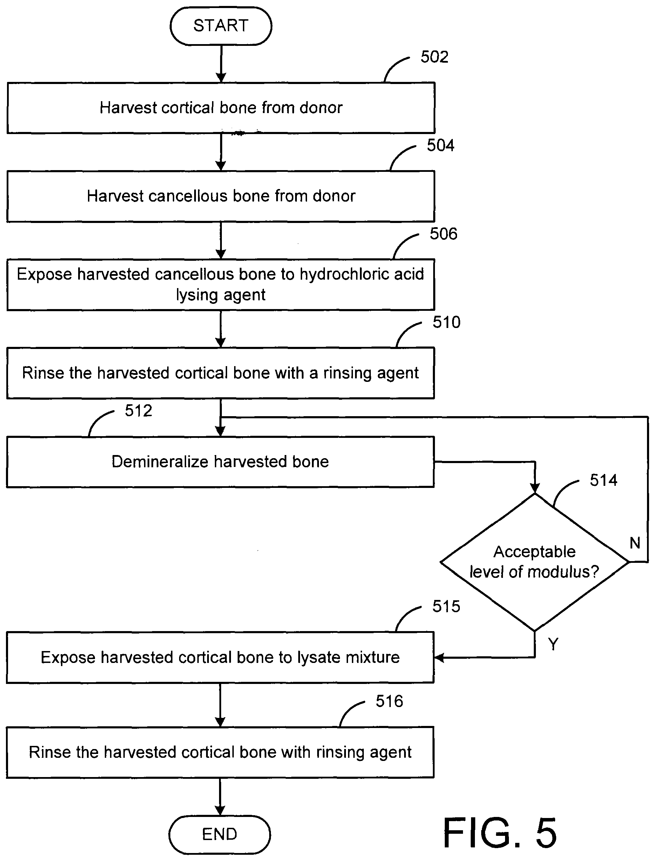

Reference is now made to FIG. 5, which depicts an alternative embodiment of the disclosure. Cortical bone is harvested from a cadaver, live donor, or harvested autologously from a patient in box 502. Depending on the application of the implant, cortical bone may be ground or cut to a desired shape and configuration. Accordingly, cancellous bone is also harvested from the same donor as the cortical bone in box 504. The cancellous bone may be ground or cut to a particular shape or configuration depending on the application of the implant. Care may be taken to retain as much cellular material, bone marrow, and/or blood within the cancellous bone during harvest and cutting operations. Cancellous bone may have cortical bone portions such as in the iliac crest, vertebral bodies, chondyles, *etc.* Accordingly, the cancellous bone may have cortical portions removed prior to further processing. The cancellous bone is exposed to a lysing agent, such as, but not limited to, hydrochloric acid in box 506 to lyse cells remaining in porous bone structure and on bone surface.

The harvested cortical bone may be cleaned and demineralized in boxes 510 and box 512 to remove its mineral content, including, but not limited to, calcium salts. This demineralization process may involve soaking in acid and/or cyclic vacuum perfusion of acid into the pores of the bone.

Employing a vacuum assisted cyclic method of demineralization may, as a non-limiting example, decrease required demineralization time from one to fifty-nine minutes. A vacuum assisted cyclic demineralization cycle can facilitate substantially uniform removal of calcium minerals throughout the implant rather than just on the surface. Non-uniform removal of calcum minerals may occur if the demineralization step is performed by soaking the cortical bone in acid. Non-uniform calcium mineral removal can result in varying calcium concentrations gradient throughout different portions of the implant.

Employing vacuum assisted cyclic demineralization can result in a more homogenous calcium concentration relative to soaking the sample in acid, resulting in stronger implants with better toughness and resilience. Additionally, this process can be used to reduce the modulus of bone to better match the natural mechanical properties found at the patient's surgical implantation site. This can be advantageous in osteoporotic, osteopenic patients, or patients with low bone density or bone mineral density. Also, this homogenous reduced modulus is advantageous in surgical sites where the implantation site is decorticated. Uniform calcium mineral removal can also reduce subsidence rates in spinal fusions. Also, better growth factor retention may be found within cortical bone using vacuum assisted cyclic demineralization.

If it is determined in box 514 that the level of modulus of the cortical bone is acceptable, this reduced modulus cortical or corticocancellous bone can also be made with binded growth factors and/or bioactive materials. The lysing of the cells of the harvested cancellous bone releases and solubilizes growth factors and bioactive materials contained in the cellular material. Hydrochloric acid also restricts the solubilized bioactives and growth factors from binding to the cancellous. The solubilized growth factors and bioactives in the hydrochloric acid are added to the cortical bone that is harvested from the same donor in box 515. The growth factors and bioactives readily bind to the mineralized or demineralized cortical bone.

The bone may be further rinsed and cleaned after the binding in box 516 to remove excess hydrochloric acid, cell fragments, lipids, and/or debris, *etc*. Rinsing solutions can be water, saline, peroxides, alcohol, crystalloids, sterilizing fluids, preserving fluids, storage agents, or other fluids used in processing of allografts. Additionally, the implant can be made flexible before or after binding bioactives and/or growth factors if the implant is further demineralized. Accordingly, reduced modulus structural bone implants with increased bioactivity may be made in this manner.

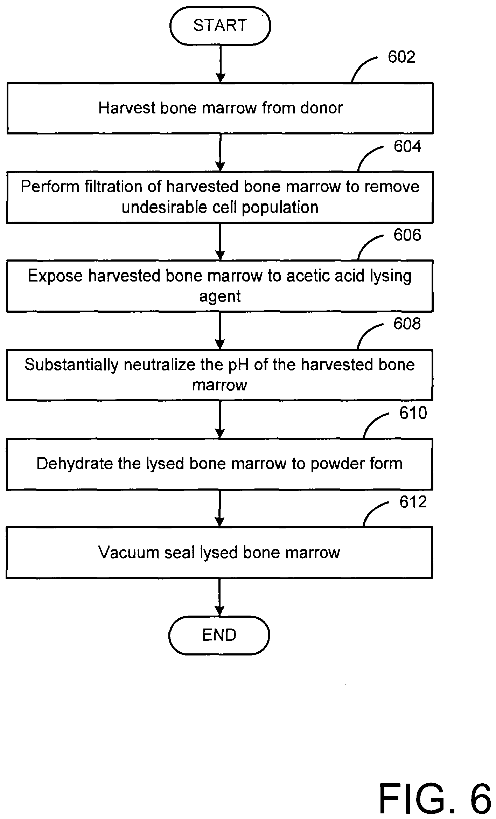

Reference is now made to FIG. 6, which depicts an alternative embodiment of the disclosure. Bone marrow is harvested from a cadaver, live donor, or harvested autologously from a patient in box 602. If a cadaver donor is used, a higher volume of marrow may be obtained by harvesting the marrow before any bone sectioning is done. In some embodiments, using a cannulated drill attached to a vacuum line to harvest marrow would also increase the yield of bone marrow from a cadaver donor. The tip of the cannulated drill breaks apart within the cancellous bone, allowing the vacuum to pull marrow through the cannula into a collection chamber.

Harvesting marrow from a living donor prior to the donor being removed from life support can also be employed as a marrow harvesting technique, because as the marrow is removed, blood flow caused by physiological circulation flushes additional bone marrow material into the area for further aspiration. After marrow has been harvested, particular cell types (such as mesenchymal stem cells, osteoblasts, osteocytes, or other progenitor cells) may be concentrated by filtration, centrifugation, magnetic bead binding, fluorescence activated cell sorting (FACS), and/or other cell sorting or concentration techniques as can be appreciated to increase the cell concentration, fractionate cell types, or eliminate particular cell types from the solution in box 604. Once, the desired cell population is obtained, it may be exposed to a lysis technique previously described, such as exposure to acetic acid in box 606.

Once acetic acid is added to the cells, they are given time to lyse and the growth factors and other bioactives are solubilized. The solution can be centrifuged or filtered to eliminated any cell fragments or cellular debris. The solution may undergo a second filtration step to remove other solid precipitates such as precipitated hemoglobin. The solution may undergo a third filtration step to concentrate the growth factors and other bioactives in the solution. The solution is then dehydrated by methods previously described, such as lyophilization. The solution is reduced to a water soluble powder in box 610 and may be sealed under vacuum to increase shelf-life in box 612. The solution can also be frozen to increase shelf life. This powder can be rich in a number or bioactive molecules and/or growth factors including, but not limited to, BMP-2, VEGF, aFGF, FGF-6, TGF-B1, and others as can be appreciated.

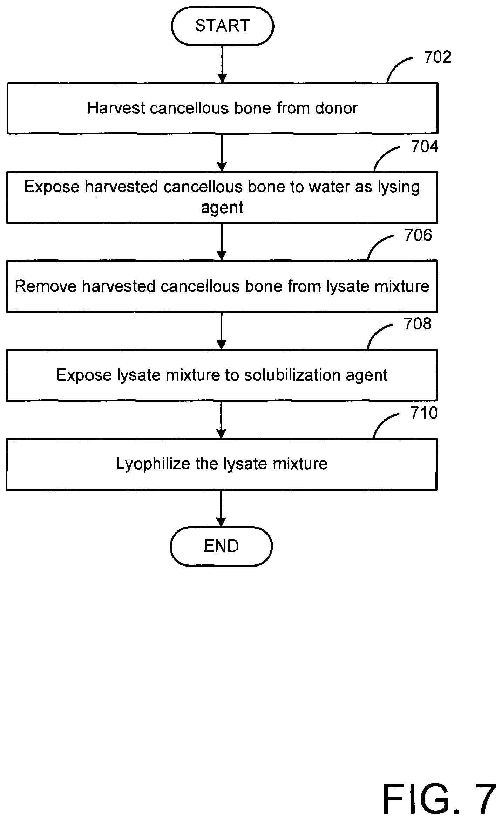

Reference is now made to FIG. 7, which depicts an alternative embodiment of the disclosure. In the depicted embodiment, cancellous bone is recovered from a cadaver, live donor, or harvested autologously from a patient in box 702. If required by a particular implant application, the harvested cancellous bone may be ground or cut to a desired shape and configuration. Care may be taken to retain as much bone marrow and blood within the bone during harvest and cutting operations. Cancellous bone may have cortical bone portions such as in the iliac crest, vertebral bodies, chondyles, *etc*. Accordingly, the cancellous bone may have cortical portions removed prior to further processing. The harvested cancellous bone is then exposed to a lysing agent, such as water, to lyse the cells contained in the cancellous bone in box 704. If a particular anticoagulant, such as heparin, is used as a lysing agent, the growth factors released by lysing the cells will be solubilized in solution. If no anticoagulant is used or if a different anticoagulant is used, such as sodium citrate, the cells will be lysed and release growth factors, but they will not be fully solubilized in the fluid.

In this case, the bone is then removed from the fluid in box 706 and a solubilization agent, such as an acid, is added to the fluid to solubilize the growth factors and other bioactives in box 708. Once the growth factors and other bioactives have been solubilized, the fluid may be neutralized and/or lyophilized in box 710. If acetic acid was used as the solubilizer, neutralization may be unnecessary as a substantial amount of acetic acid will vaporize during lyophilization. Alternatively, other lysing agents and solubilizers could be used to lyse the cells and solubilize the growth factors, preventing the growth factors and bioactive materials from binding to the cancellous bone from which the cells were harvested.

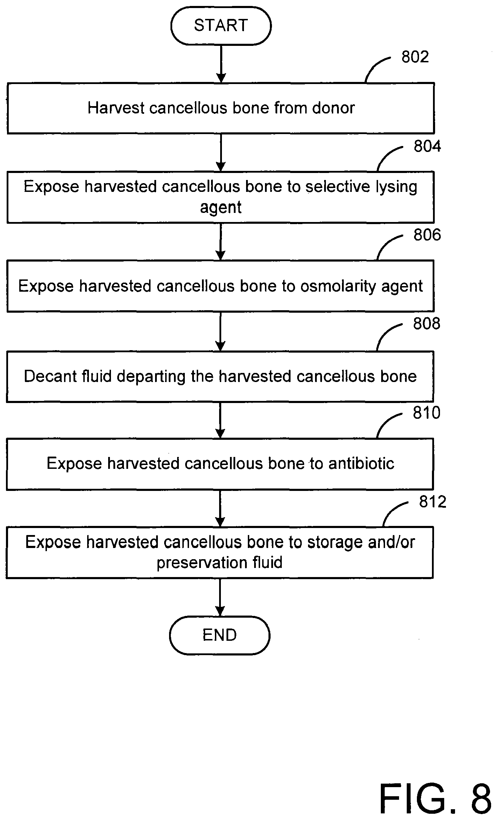

Reference is now made to FIG. 8, which depicts an alternative embodiment of the disclosure. In the depicted embodiment, cancellous bone is recovered from a cadaver, live donor, or harvested autologously from a patient in box 802. If required by a particular implant application, cancellous bone may be ground or cut to a desired shape and configuration. Care may be taken to retain as much bone marrow and blood within the bone during harvest and cutting operations. Cancellous bone may have cortical bone portions such as in the iliac crest, vertebral bodies, chondyles, *etc*. Accordingly, cortical portions of the harvested cancellous bone may be removed. The harvested cancellous bone is exposed to water to selectively lyse undesired cells types such as red blood cells, white blood cells, *etc* in box 804. In some embodiments, ratios of bone to water from 1 part bone to 1 part water and ranging to 1 part bone to 200 parts water can be employed. Any remaining viable cells that are not attached to the bone may be rinsed away in this fashion. Additionally, using a weak lysing agent (such as less then 1M acetic acid) may result in binding solubilized growth factors to the bone but still retaining viable progenitor cells attached to the bone.

The desired cells, such as mesenchymal stem cells, bone marrow stromal cells, progenitor cells, *etc.,* remain viable in porous bone structure and on bone surface. Other mechanical lysing techniques previously decribed, such as sonication, stirring induced shear, thermoslysis, *etc.,* may be used in conjunction with the water bath to facilitate lysing of cellular material. After a lysing time (*e.g.,* 1 minute - 50 hours) has elapsed, saline is added to return osmolarity of the solution to physiological levels (*e.g.,* approximately 0.9% salt) in box 806. After the solution is returned to isotonic conditions, the fluid is decanted leaving the bone in box 808. The effective rinse also facilitates removal of undesired cells unattached to the cancellous bone and discards them in the decanting step.

Antibiotics may be applied to the bone in box 810 to help with decreasing bioburden levels. Alternatively, in some embodiments antibiotics can be administered to the harvested cancellous bone prior to the lysing step. Some antibiotics that may be used include gentamicin, vancomycin, amphotericin, other antibiotics previously mentioned or as can be appreciated, or various antibiotics that can be used to reduce bioburden in allograft tissues. After the reduction of bioburden, the bone may be exposed to storage or preservation fluids such as DMEM, DMSO, sucrose, mannitol, glucose, *etc*., in box 812. The bone is then frozen until thawed for use in a surgical procedure to repair a skeletal defect. In some embodiments, the bone can be frozen at temperatures at or below -40 C.

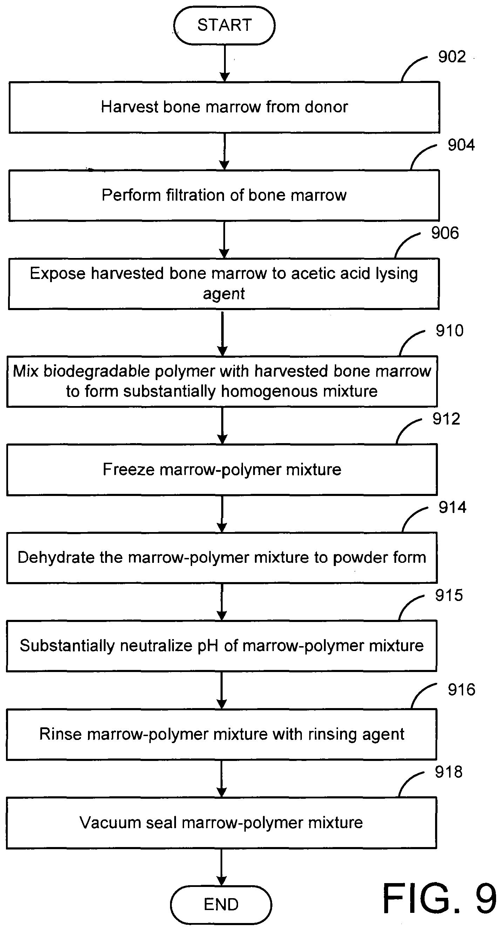

Reference is now made to FIG. 9, which depicts an alternative embodiment of the disclosure. In the depicted embodiment, the growth factors and bioactives obtained in the embodiments described above with reference to FIGS. 6 and/or 7 (as a non-limiting example) may be added to a biodegradable or resorbable polymer prior to dehydration. Accordingly, bone marrow harvested in box 902 can be subjected to at least one filtration process in box 904 as described above with reference to FIG. 6. The harvested bone marrow can be subjected to a lysing agent in box 906 as also described above.

In this embodiment, the growth factors and bioactives are harvested as previously described and added to a polymer with a common solvent, such as an acid. The biodegradable polymer may be a protein or polysaccharide, such as collagen, hyaluronan, chitosan, gelatin, *etc.,* and combinations of two or more polymers. After the growth factors and bioactives are added to the polymer, it is mixed to obtain a substantially homogenous solution in box 910. Any bubbles or impurities may then be removed from the substantially homogenous solution. If other materials (such as, but not limited to, calcium phosphate, demineralized bone, hydroxyapatite, heparin, chondroitin sulfate, *etc*.) are desired to be embedded into the implant for growth factor attachment, degradation by products, and/or mechanical reinforcement, they can also be added to the mixture.

The mixture is frozen in box 912 at a temperature that can range, in some embodiments, from -200 C to 0 C, to nucleate the water contained in the mixture into ice as well as condense the polymer/bioactive mixture into a porous structure. The mixture can be frozen in any geometry including, spherical, cylindrical, rectangular, in sheet form, tube form, *etc.* The implant will tend to retain this shape with its shape memory properties of the polymer is given space to expand in vivo. Temperatures can be increased to create larger pores or decreased to create small pores. Pores can be made directional by locating the cold temperature source substantially perpendicularly to the desired direction of the pores. Once the mixture is frozen at the desired temperature and pore direction, the implant is lyophilized and/or dehydrated in box 914 to substantially eliminate the water contained within it. If acetic acid or another volatile substance was used as the solvent, that solvent will also be substantiailly eliminated by lyophilization.

After the lyophilization cycle is complete, the scaffold may be substantially neutralized in ethanol, saline, base, or buffer depending on the solvent used as a lysing agent in box 915. In the case of an acetic acid solvent, the lyophilized implant may be rinsed in ethanol followed by saline or other rinsing agent in box 916. After the saline rinse, the implant may be rinsed free of salts with water and vacuum dried or lyophilized to extend shelf-life. The dehydrated implants may be packaged under vacuum or sealed in vacuum sealed vials in box 918. The implant can also be compressed prior to freezing and lyophilization or after neutralization and lyophilization to create a compacted scaffold that expands when exposed to fluid. Upon exposure to fluid, such an implant expands to substantially to approximately the original scaffold size. Delayed expansion may be achieved by compressing the neutralized scaffold and drying without freezing.

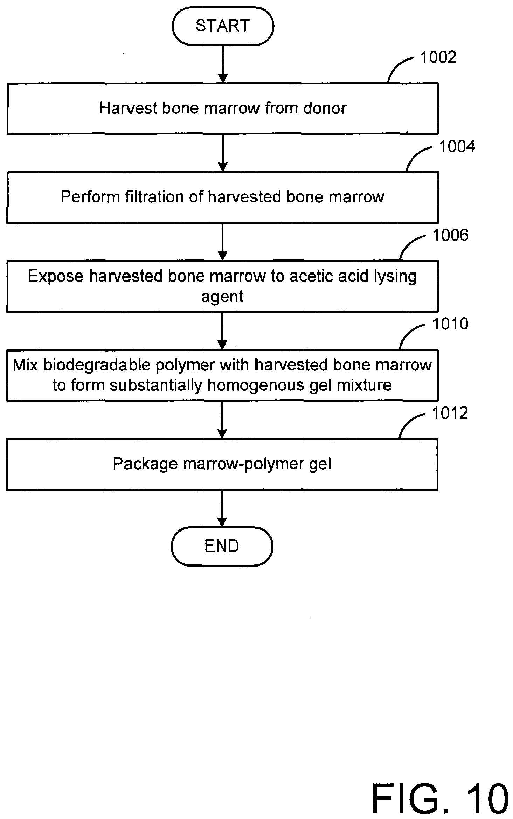

Reference is now made to FIG. 10, which depicts an alternative embodiment of the disclosure. In the depicted embodiment, the growth factors and/or bioactives obtained in the embodiments discussed with reference FIGS. 6 and 7 (as a non-limiting example) may be added to a biodegradable or resorbable polymer to create a flowable fluid and/or gel. In this embodiment, the growth factors and bioactives are harvested as previously described and added to a polymer with a common solvent, such as an acid. Accordingly, bone marrow harvested in box 1002 can be subjected to at least one filtration process in box 1004 as described above with reference to FIG. 6. The harvested bone marrow can be subjected to a lysing agent in box 1006 as also described above.

The biodegradable polymer may be a protein or polysaccharide, such as collagen, hyaluronan, chitosan, gelatin, *etc.,* and combinations of two or more polymers. After the growth factors and bioactives are added to the polymer, it is mixed to obtain a substantially homogenous solution in box 1010. Any bubbles or impurities may be removed. If other materials (including, but not limited to, calcium phosphate, demineralized bone, hydroxyapatite, heparin, chondroitin sulfate, *etc*.) are desired to be embedded into the implant for growth factor attachment, degradation by products, and/or mechanical reinforcement, they can also be added to the mixture.

A lysing agent can be chosen that is well tolerated by the body. For example, the growth factors and bioactives can be added to chitosan and in an acetic acid solution (0.01 M - 17M). Demineralized bone can also be added to the solution. The solution is mixed, and bubbles can be removed by applying vacuum or centrifugation. The gel can be packaged in syringes and either frozen and/or kept at ambient temperature in box 1012. Once injected and/or implanted into the body, the gel binds to tissue. Physiological fluids may buffer the gel to neutralize the pH and cause the gel to solidify in situ. Once the gel solidifies, the desired therapeutic implant remains in the intended surgical site and minimizes migration.

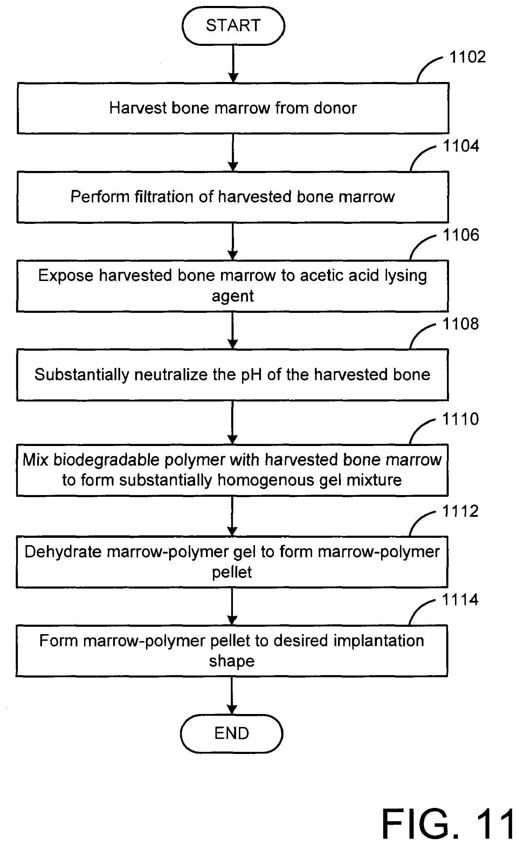

Reference is now made to FIG. 11, which depicts an alternative embodiment of the disclosure. A gel obtained as described in the above embodiment discussed with reference to FIG. 10 may be dehydrated using techniques such as vacuum drying, solvent evaporation, *etc.,* to reduce the gel into a semi-rigid film and/or pellet. Accordingly, bone marrow harvested in box 1102 can be subjected to at least one filtration process in box 1104 as described above with reference to FIG. 6. The harvested bone marrow can be subjected to a lysing agent in box 1106 as also described above.

The gel is dehydrated as described above in box 1112. The pellets may be ground further or cut into the desired particle size depending on a desired implant application in box 1114. Once exposed to fluid and implanted into the surgical site, the pellets and/or powder resulting from ground pellets form a cohesive putty that can also bind to tissue. This binding property keeps the putty substantially in place at the surgical site when implanted. This putty can be used as a bioactive surgical adhesive. The application of such a putty may also be advantageous when used with autologous materials used in surgical procedures, such as autograft bone used in spinal fusion procedures, because it may be beneficial to help keep the autograft in a cohesive mass and minimize migration.

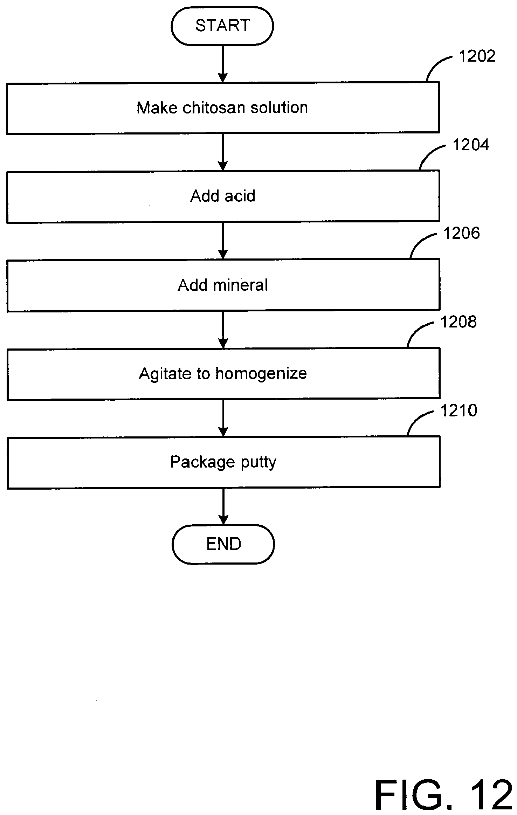

Referring now to FIG. 12, shown is a flow diagram illustrating a method to produce an embodiment of a low pH chitosan/mineral putty. In box 1202, a chitosan solution is made. The chitosan solution may be in the range of about 1% to about 25%. An acid (*e.g*., acetic acid) is then added in box 1204 to put the solution into a suspension. The acid may be in the range of about 0.1% to about 25%. A mineral in powder or granular form is then added in box 1206 and agitated to a homogenous mixture in box 1208. The putty is then packaged either wet or frozen in box 1210.

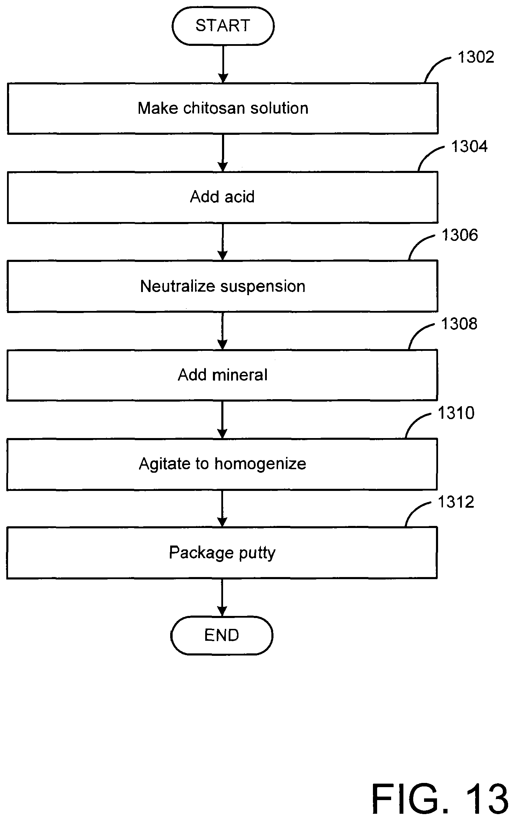

Referring next to FIG. 13, shown is a flow diagram illustrating a method to produce an embodiment of a neutral to partially neutral chitosan/mineral putty. In box 1302, a chitosan solution is made. The chitosan solution may be in the range of about 1% to about 25%. An acid (*e.g*., acetic acid) is then added in box 1304 to put the solution into a suspension. The acid may be in the range of about 0.1% to about 25%. The suspension is then neutralized or partially neutralized in box 1306 by adding base solution (*e.g*., sodium hydroxide or ammonium hydroxide) and agitating to homogenize the base solution. A mineral in powder or granular form is then added in box 1308 and agitated to a homogenous mixture in box 1310. The putty is then packaged either wet or frozen in box 1312.

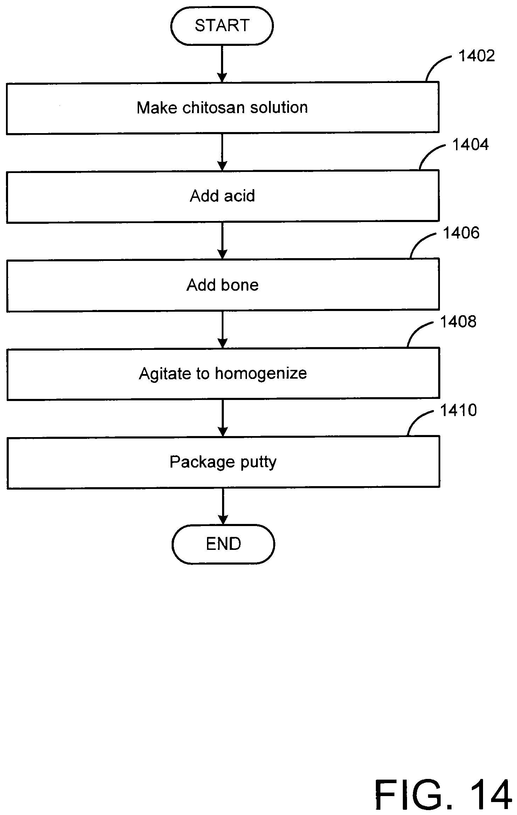

Reference is now made to FIG. 14, which depicts a flow diagram illustrating a method to produce an embodiment of a low pH chitosan/bone putty. In box 1402, a chitosan solution is made. The chitosan solution may be in the range of about 1% to about 25%. An acid (*e.g*., acetic acid) is then added in box 1404 to put the solution into a suspension. The acid may be in the range of about 0.1% to about 25%. Bone in powder or granular form is then added in box 1406 and agitated to a homogenous mixture in box 1408. The putty is then packaged either wet or frozen in box 1410.

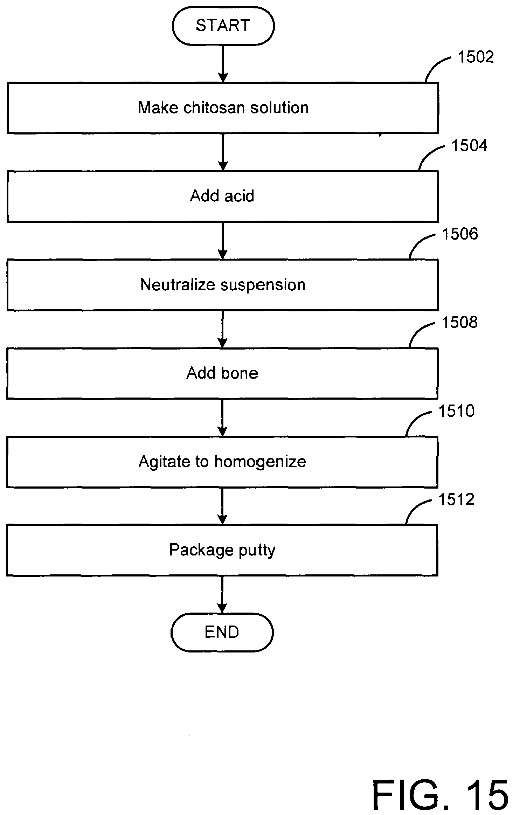

Referring now to FIG. 15, shown is a flow diagram illustrating a method to produce an embodiment of a neutral to partially neutral chitosan/bone putty. In box 1502, a chitosan solution is made. The chitosan solution may be in the range of about 1% to about 25%. An acid (*e.g*., acetic acid) is then added in box 1504 to put the solution into a suspension. The acid may be in the range of about 0.1% to about 25%. The suspension is then neutralized or partially neutralized in box 1506 by adding base solution (*e.g*., sodium hydroxide or ammonium hydroxide) and agitating to homogenize the base solution. Bone (*e.g*., allograft bone) in powder or granular form is then added in box 1508 and agitated to a homogenous mixture in box 1510. The putty is then packaged either wet or frozen in box 1512.

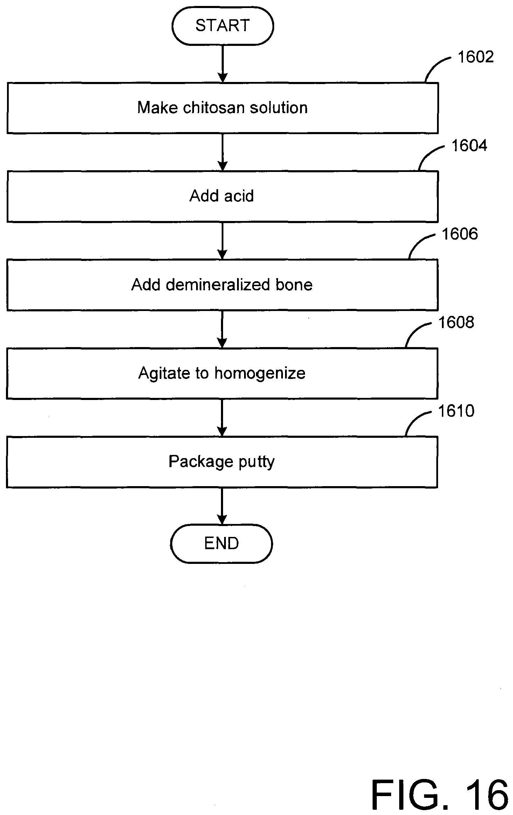

Referring next to FIG. 16, shown is a flow diagram illustrating a method to produce an embodiment of a low pH chitosan/demineralized bone putty. In box 1602, a chitosan solution is made. The chitosan solution may be in the range of about 1% to about 25%. An acid (*e.g*., acetic acid) is then added in box 1604 to put the solution into a suspension. The acid may be in the range of about 0.1% to about 25%. Demineralized or partially demineralized bone in powder or granular form is then added in box 1606 and agitated to a homogenous mixture in box 1608. The putty is then packaged either wet or frozen in box 1610.

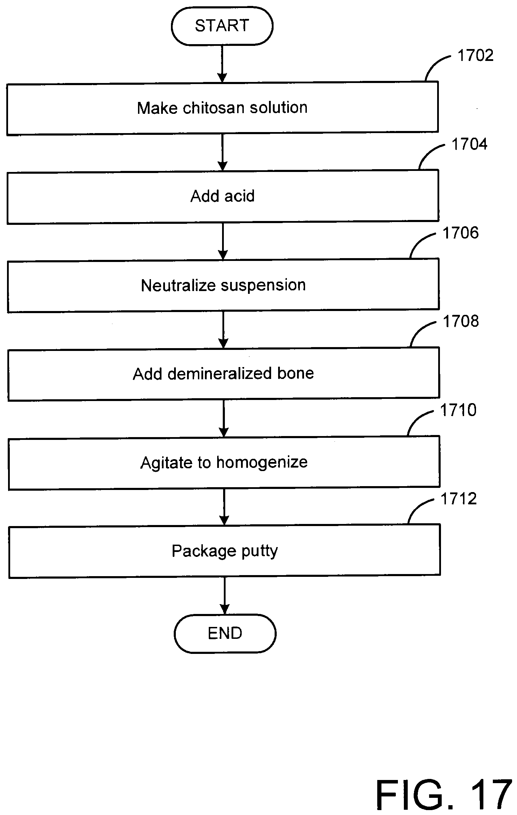

Reference is now made to FIG. 17, which depicts a flow diagram illustrating a method to produce an embodiment of a neutral to partially neutral chitosan/demineralized bone putty. In box 1702, a chitosan solution is made. The chitosan solution may be in the range of about 1% to about 25%. An acid (*e.g*., acetic acid) is then added in box 1604 to put the solution into a suspension. The acid may be in the range of about 0.1% to about 25%. The suspension is then neutralized or partially neutralized in box 1606 by adding base solution (*e.g*., sodium hydroxide or ammonium hydroxide) and agitating to homogenize the base solution. Demineralized or partially demineralized bone (*e.g*., allograft bone) in powder or granular form is then added in box 1708 and agitated to a homogenous mixture in box 1710. The putty is then packaged either wet or frozen in box 1712.

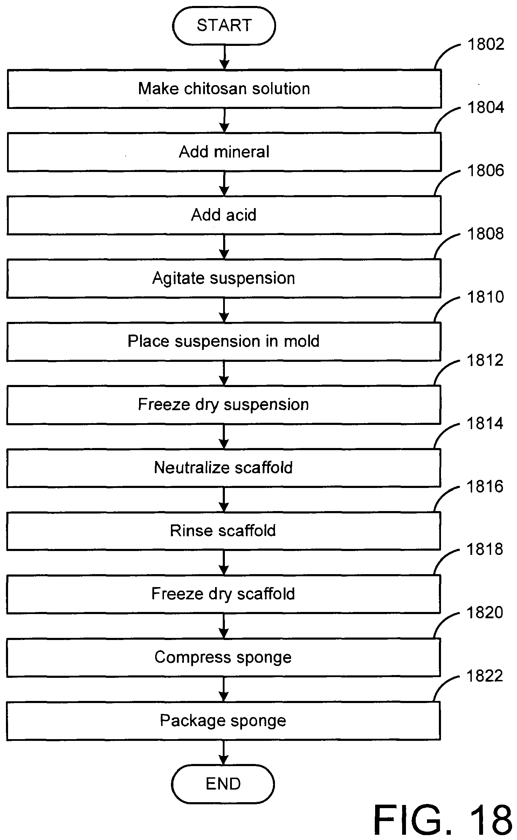

Referring now to FIG. 18, shown is a flow diagram illustrating a method to produce an embodiment of a neutral or partially neutral chitosan/mineral scaffold sponge. In box 1802, a chitosan solution is made. The chitosan solution may be in the range of about 1% to about 25%. A mineral in powder or granular form is then added in box 1804 and agitated to a homogenous mixture. An acid (*e.g.*, acetic acid) is then added in box 1806 to put the solution into a suspension and agitated in box 1808. The acid may be in the range of about 0.1% to about 25%. The suspension is then placed into molds in box 1810 to conform to one or more desired shapes. The suspension is then freeze dried in box 1812. The molds are placed into a freezer and the suspensions are frozen to allow crystal formation. The frozen suspensions are lyophilized and the formed scaffolds are pulled out of molds. The scaffolds are then neutralized or partially neutralized in box 1814 by soaking in a base solution (*e.g*., sodium hydroxide or ammonium hydroxide). The scaffolds are then rinsed of any remaining base solution in sterile water or PBS in box 1816 and freeze dried in box 1818 where the scaffolds are frozen and lyophilized. The scaffolds are compressed into the desired shape in box 1820 and packaged and sterilized in box 1822.

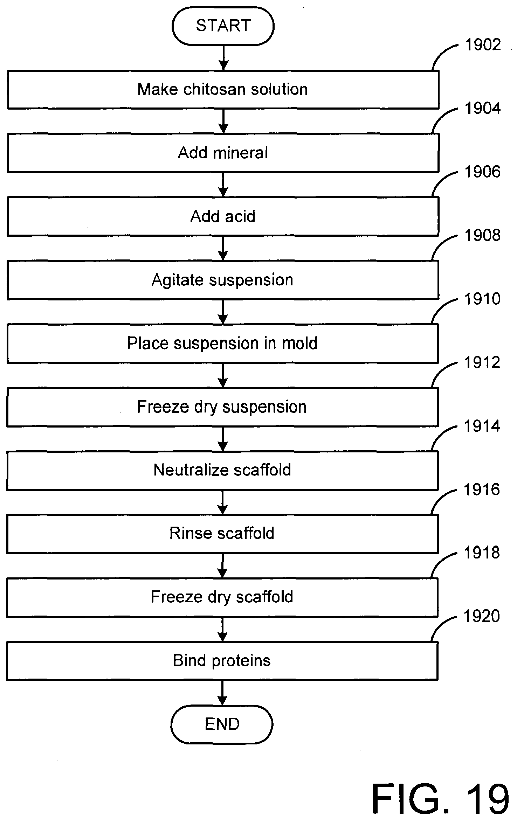

Referring next to FIG. 19, shown is a flow diagram illustrating a method to produce another embodiment of a neutral or partially neutral chitosan/mineral scaffold sponge. In box 1902, a chitosan solution is made. The chitosan solution may be in the range of about 1% to about 25%. A mineral in powder or granular form is then added in box 1904 and agitated to a homogenous mixture. An acid (*e.g*., acetic acid) is then added in box 1906 to put the solution into a suspension and agitated in box 1908. The acid may be in the range of about 0.1% to about 25%. The suspension is then placed into molds in box 1910 to conform to one or more desired shapes. The suspension is then freeze dried in box 1912. The molds are placed into a freezer and the suspensions are frozen to allow crystal formation. The frozen suspensions are lyophilized and the formed scaffolds are pulled out of molds. The scaffolds are then neutralized or partially neutralized in box 1914 by soaking in a base solution (*e.g*., sodium hydroxide or ammonium hydroxide). The scaffolds are then rinsed of any remaining base solution in sterile water or PBS in box 1916 and freeze dried in box 1918 where the scaffolds are frozen and lyophilized. Proteins are then bound onto the scaffold by way of soaking or vacuum perfusion in box 1920.

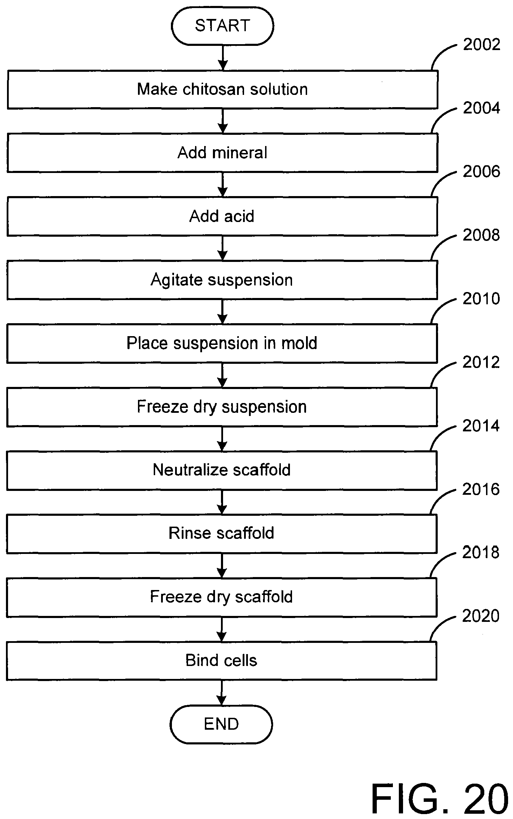

Reference is now made to FIG. 20, which depicts a flow diagram illustrating a method to produce an embodiment of a neutral or partially neutral chitosan/mineral scaffold sponge including seed cells. In box 2002, a chitosan solution is made. The chitosan solution may be in the range of about 1% to about 25%. A mineral in powder or granular form is then added in box 2004 and agitated to a homogenous mixture. An acid (*e.g*., acetic acid) is then added in box 2006 to put the solution into a suspension and agitated in box 2008. The acid may be in the range of about 0.1% to about 25%. The suspension is then placed into molds in box 2010 to conform to one or more desired shapes. The suspension is then freeze dried in box 2012. The molds are placed into a freezer and the suspensions are frozen to allow crystal formation. The frozen suspensions are lyophilized and the formed scaffolds are pulled out of molds. The scaffolds are then neutralized or partially neutralized in box 2014 by soaking in a base solution (*e.g*., sodium hydroxide or ammonium hydroxide). The scaffolds are then rinsed of any remaining base solution in sterile water or PBS in box 2016 and freeze dried in box 2018 where the scaffolds are frozen and lyophilized. Seed cells are then bound onto the scaffold by way of hydration, soaking or vacuum perfusion in box 2020.

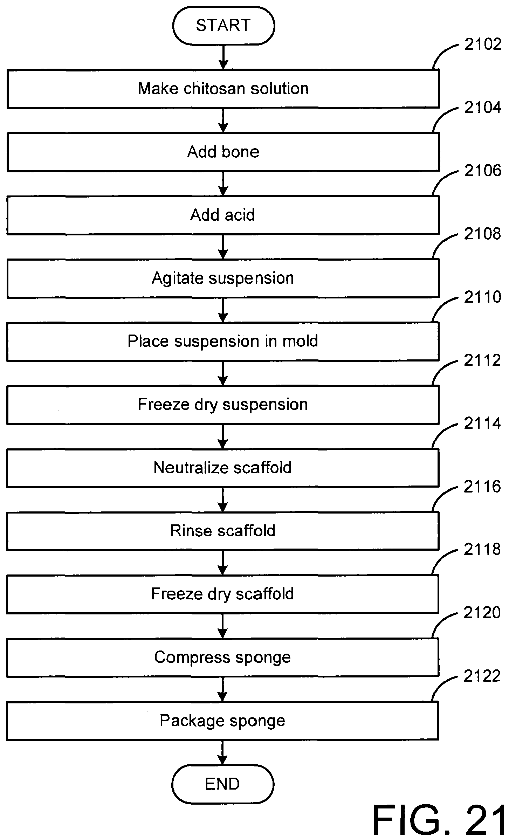

Referring now to FIG. 21, shown is a flow diagram illustrating a method to produce an embodiment of a neutral or partially neutral chitosan/bone scaffold sponge. In box 2102, a chitosan solution is made. The chitosan solution may be in the range of about 1% to about 25%. Bone in powder or granular form is then added in box 2104 and agitated to a homogenous mixture. An acid (*e.g*., acetic acid) is then added in box 2106 to put the solution into a suspension and agitated in box 2108. The acid may be in the range of about 0.1% to about 25%. The suspension is then placed into molds in box 2110 to conform to one or more desired shapes. The suspension is then freeze dried in box 2112. The molds are placed into a freezer and the suspensions are frozen to allow crystal formation. The frozen suspensions are lyophilized and the formed scaffolds are pulled out of molds. The scaffolds are then neutralized or partially neutralized in box 2114 by soaking in a base solution (*e.g*., sodium hydroxide or ammonium hydroxide). The scaffolds are then rinsed of any remaining base solution in sterile water or PBS in box 2116 and freeze dried in box 2118 where the scaffolds are frozen and lyophilized. The scaffolds are compressed into the desired shape in box 2120 and packaged and sterilized in box 2122.

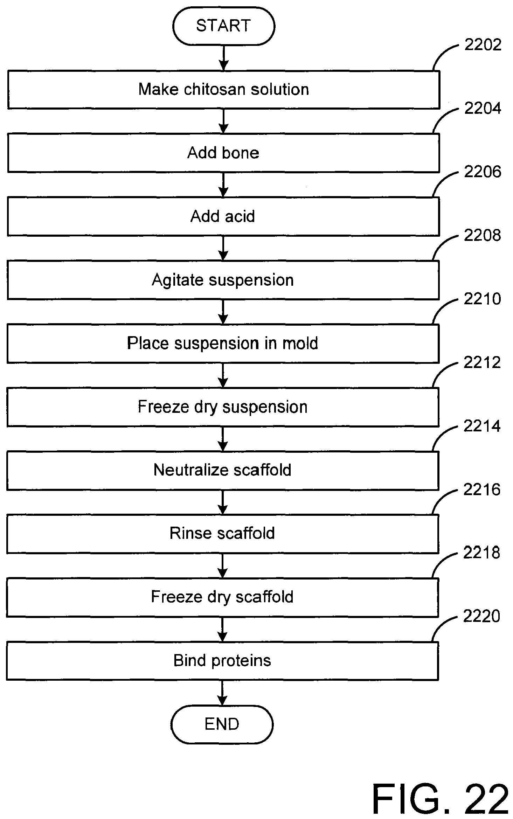

Referring next to FIG. 22, shown is a flow diagram illustrating a method to produce another embodiment of a neutral or partially neutral chitosan/bone scaffold sponge. In box 2202, a chitosan solution is made. The chitosan solution may be in the range of about 1% to about 25%. Bone in powder or granular form is then added in box 2204 and agitated to a homogenous mixture. An acid (*e.g*., acetic acid) is then added in box 2206 to put the solution into a suspension and agitated in box 2208. The acid may be in the range of about 0.1% to about 25%. The suspension is then placed into molds in box 2210 to conform to one or more desired shapes. The suspension is then freeze dried in box 2212. The molds are placed into a freezer and the suspensions are frozen to allow crystal formation. The frozen suspensions are lyophilized and the formed scaffolds are pulled out of molds. The scaffolds are then neutralized or partially neutralized in box 2214 by soaking in a base solution (*e.g*., sodium hydroxide or ammonium hydroxide). The scaffolds are then rinsed of any remaining base solution in sterile water or PBS in box 2216 and freeze dried in box 2218 where the scaffolds are frozen and lyophilized. Proteins are then bound onto the scaffold by way of soaking or vacuum perfusion in box 2220.

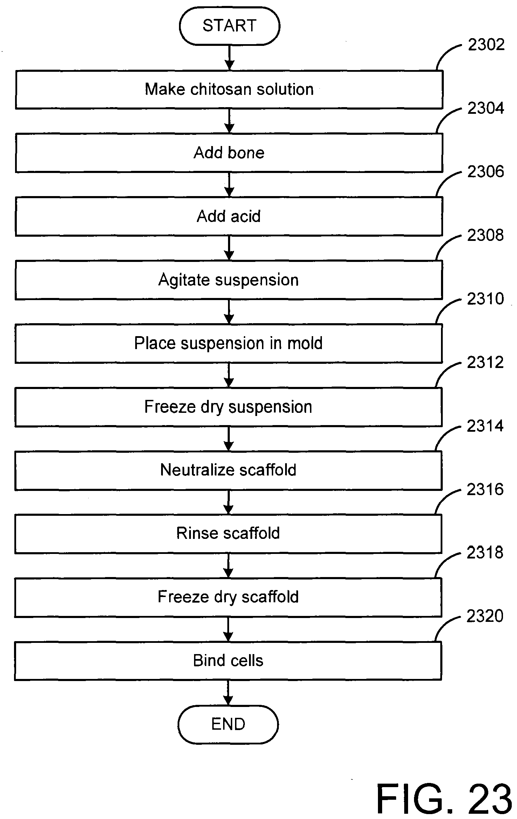

Reference is now made to FIG. 23, which depicts a flow diagram illustrating a method to produce an embodiment of a neutral or partially neutral chitosan/bone scaffold sponge including seed cells. In box 2302, a chitosan solution is made. The chitosan solution may be in the range of about 1% to about 25%. Bone in powder or granular form is then added in box 2304 and agitated to a homogenous mixture. An acid (*e.g*., acetic acid) is then added in box 2306 to put the solution into a suspension and agitated in box 2308. The acid may be in the range of about 0.1% to about 25%. The suspension is then placed into molds in box 2310 to conform to one or more desired shapes. The suspension is then freeze dried in box 2312. The molds are placed into a freezer and the suspensions are frozen to allow crystal formation. The frozen suspensions are lyophilized and the formed scaffolds are pulled out of molds. The scaffolds are then neutralized or partially neutralized in box 2314 by soaking in a base solution (*e.g*., sodium hydroxide or ammonium hydroxide). The scaffolds are then rinsed of any remaining base solution in sterile water or PBS in box 2316 and freeze dried in box 2318 where the scaffolds are frozen and lyophilized. Seed cells are then bound onto the scaffold by way of hydration, soaking or vacuum perfusion in box 2320.

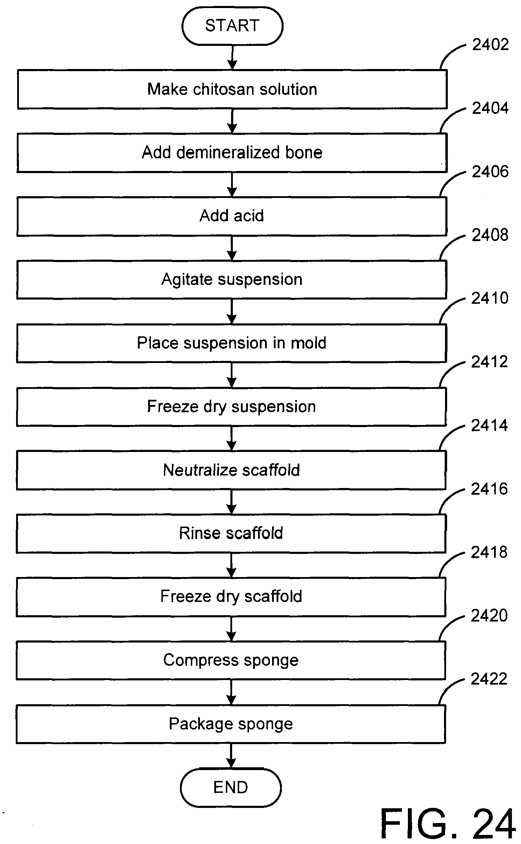

Referring now to FIG. 24, shown is a flow diagram illustrating a method to produce an embodiment of a neutral or partially neutral chitosan/demineralized bone scaffold sponge. In box 2402, a chitosan solution is made. The chitosan solution may be in the range of about 1% to about 25%. Demineralized or partially demineralized bone in powder or granular form is then added in box 2404 and agitated to a homogenous mixture. An acid (e.g., acetic acid) is then added in box 2406 to put the solution into a suspension and agitated in box 2408. The acid may be in the range of about 0.1% to about 25%. The suspension is then placed into molds in box 2410 to conform to one or more desired shapes. The suspension is then freeze dried in box 2412. The molds are placed into a freezer and the suspensions are frozen to allow crystal formation. The frozen suspensions are lyophilized and the formed scaffolds are pulled out of molds. The scaffolds are then neutralized or partially neutralized in box 2414 by soaking in a base solution (e.g., sodium hydroxide or ammonium hydroxide). The scaffolds are then rinsed of any remaining base solution in sterile water or PBS in box 2416 and freeze dried in box 2418 where the scaffolds are frozen and lyophilized. The scaffolds are compressed into the desired shape in box 2420 and packaged and sterilized in box 2422.

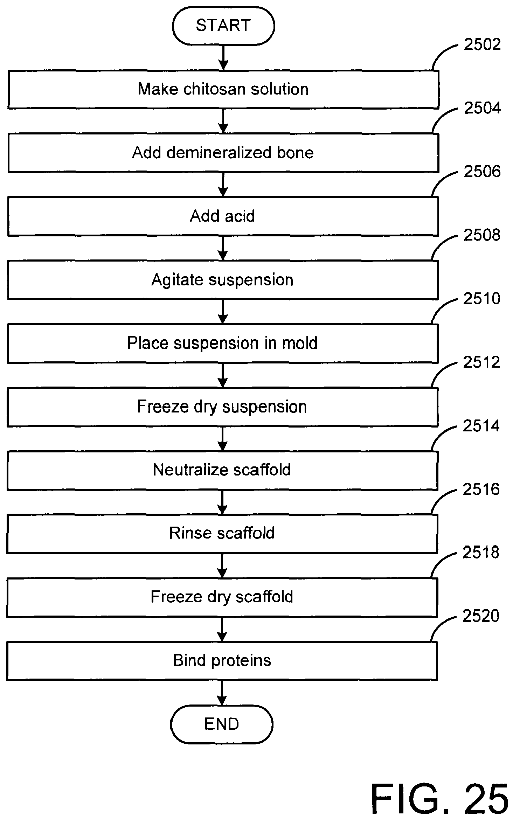

Referring next to FIG. 25, shown is a flow diagram illustrating a method to produce another embodiment of a neutral or partially neutral chitosan/demineralized bone scaffold sponge. In box 2502, a chitosan solution is made. The chitosan solution may be in the range of about 1% to about 25%. Demineralized or partially demineralized bone in powder or granular form is then added in box 2504 and agitated to a homogenous mixture. acid (*e.g*., acetic acid) is then added in box 2506 to put the solution into a suspension and agitated in box 2508. The acid may be in the range of about 0.1% to about 25%. The suspension is then placed into molds in box 2510 to conform to one or more desired shapes. The suspension is then freeze dried in box 2512. The molds are placed into a freezer and the suspensions are frozen to allow crystal formation. The frozen suspensions are lyophilized and the formed scaffolds are pulled out of molds. The scaffolds are then neutralized or partially neutralized in box 2514 by soaking in a base solution (*e.g*., sodium hydroxide or ammonium hydroxide). The scaffolds are then rinsed of any remaining base solution in sterile water or PBS in box 2516 and freeze dried in box 2518 where the scaffolds are frozen and lyophilized. Proteins are then bound onto the scaffold by way of soaking or vacuum perfusion in box 2520.

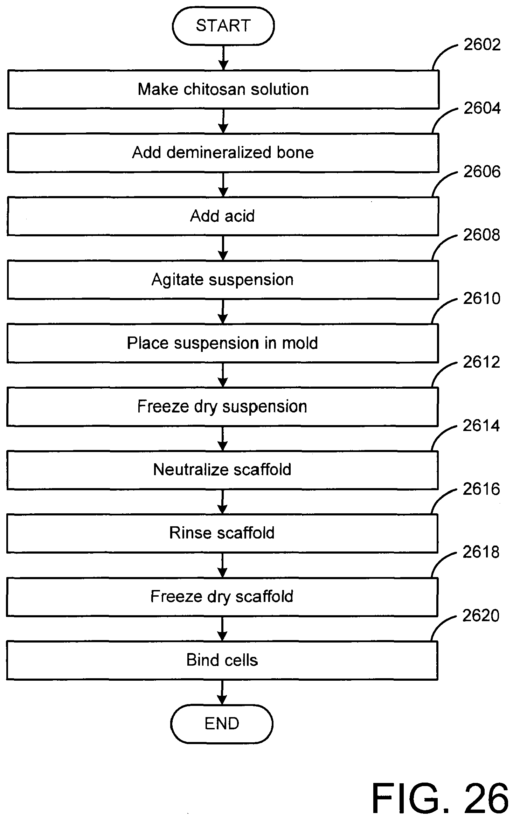

Reference is now made to FIG. 26, which depicts a flow diagram illustrating a method to produce an embodiment of a neutral or partially neutral chitosan/demineralized bone scaffold sponge including seed cells. In box 2602, a chitosan solution is made. The chitosan solution may be in the range of about 1% to about 25%. Demineralized or partially demineralized bone in powder or granular form is then added in box 2604 and agitated to a homogenous mixture. An acid (*e.g*., acetic acid) is then added in box 2606 to put the solution into a suspension and agitated in box 2608. The acid may be in the range of about 0.1% to about 25%. The suspension is then placed into molds in box 2610 to conform to one or more desired shapes. The suspension is then freeze dried in box 2612. The molds are placed into a freezer and the suspensions are frozen to allow crystal formation. The frozen suspensions are lyophilized and the formed scaffolds are pulled out of molds. The scaffolds are then neutralized or partially neutralized in box 2614 by soaking in a base solution (*e.g*., sodium hydroxide or ammonium hydroxide). The scaffolds are then rinsed of any remaining base solution in sterile water or PBS in box 2616 and freeze dried in box 2618 where the scaffolds are frozen and lyophilized. Seed cells are then bound onto the scaffold by way of hydration, soaking or vacuum perfusion in box 2620. Once the cells are bound, the scaffolds may be packaged with a cryopreservative and frozen.

The following non-limiting examples are provided for further illustration.

### Scaffold sponge formulation - percent by mass (parts/100 parts)

In a first non-limiting example, a solution of 6% of greater than 300 kDa molecular weight chitosan solution (>75% deacetylation) mixed in with 6% of tri-calcium phosphate (TCP) in 83.6% water was initially created. The solution was then mixed in with 4.4% of acetic acid to put the solution into suspension. The suspension was then placed into molds and frozen at a controlled rate by a ramp of 5°C every 15 minutes to a temperature of -80°C. Once the suspension turned to a solid, the molds were lyophilized until drying was completed. The scaffolds were then hydrated with a 2 molar NaOH solution. Scaffolds were then rinsed with sterile water until reaching a neutral pH. Scaffolds were then frozen at a controlled rate and freeze dried to until dry.

In a second non-limiting example, a solution of 4% of greater than 300 kDa molecular weight chitosan solution (>75% deacetylation) mixed in with 6% of TCP in 85.6% water was initially created. The solution was then mixed in with 4.5% of acetic acid to put the solution into suspension. The suspension was then placed into molds and frozen at a controlled rate by a ramp of 5°C every 15 minutes to a temperature of -80°C. Once the suspension turned to a solid, the molds were lyophilizeduntil drying was completed. The scaffolds were then hydrated with a 2 molar NaOH solution. Scaffolds were then rinsed with sterile water until reaching a neutral pH. Scaffolds were then frozen at a controlled rate and freeze dried to till dry

In a third non-limiting example, a solution of 3% of greater than 300 kDal molecular weight chitosan solution (>75% deacetylation) mixed in with 6% parts of TCP in 86.45% water was initially created. The solution was then mixed in with 4.55% of acetic acid to put the solution into suspension. The suspension was then placed into molds and frozen at a controlled rate by a ramp of 5°C every 15 minutes to a temperature of -80°C. Once the suspension turned to a solid, the molds were lyophilized until drying was completed. The scaffolds were then hydrated with a 2 molar NaOH solution. Scaffolds were then rinsed with sterile water until reaching a neutral pH. Scaffolds were then frozen at a controlled rate and freeze dried to until dry.

In a fourth non-limiting example, a solution of 2% of greater than 300 kDa molecular weight chitosan solution(>75% deacetylation) mixed in with 6% of TCP in 87.4% water was initially created. The solution was then mixed in with 4.6% of acetic acid to put the solution into suspension. The suspension was then placed into molds and frozen at a controlled rate by a ramp of 5°C every 15 minutes to a temperature of -80°C. Once the suspension turned to a solid, the molds were lyophilized unitl drying was completed. The scaffolds are then hydrated with a 2 molar NaOH solution. Scaffolds were then rinsed with sterile water until reaching a neutral pH. Scaffolds are then frozen at a controlled rate and freeze dried to until dry.

### Sponge formulation with protein - percent by mass (parts/100 parts)

In a non-limiting example, a solution of 3% of greater than 300 kDa molecular weight chitosan solution (>75% deacetylation) mixed in with 6% parts of TCP in 86.45% water was initially created. The solution was then mixed in with 4.55% of acetic acid to put the solution into suspension. The suspension was then placed into molds and frozen at a controlled rate by a ramp of 5°C every 15 minutes to a temperature of -80°C. Once the suspension turned to a solid, the molds were lyophilized until drying was completed. The scaffolds were then hydrated with a 2 molar NaOH solution. Scaffolds were then rinsed with sterile water until reaching a neutral pH. Scaffolds were then frozen at a controlled rate and freeze dried to until dry. The scaffolds were then fully saturated with protein solution.

### Sponge formulation with cells - percent by mass (parts/100 parts)

In a non-limiting example, a solution of 3% of greater than 300 kDa molecular weight chitosan solution (>75% deacetylation) mixed in with 6% parts of TCP in 86.45% water was initially created. The solution was then mixed in with 4.55% of acetic acid to put the solution into suspension. The suspension was then placed into molds and frozen at a controlled rate by a ramp of 5°C every 15 minutes to a temperature of -80°C. Once the suspension turned to a solid, the molds were lyophilized until drying was completed. The scaffolds were then hydrated with a 2 molar NaOH solution. Scaffolds were then rinsed with sterile water until reaching a neutral pH. Scaffolds were then frozen at a controlled rate and freeze dried to until dry. The scaffolds were then fully saturated with a physiological fluid containing viable cells.

### Acidic putty formulation - percent by mass (parts/100 parts)

In a first non-limiting example, a solution of 1% of greater than 300 kDa molecular weight chitosan solution (>75% deacetylation) mixed in 45% water was initially created. The solution was then mixed in with 1% of acetic acid to put the solution into suspension. 53% of TCP was then added into the suspension and agitated until a homogeneous mixture was reached.

In a second non-limiting example, a solution of 1% of greater than 300 kDa molecular weight chitosan solution (>75% deacetylation) mixed in 44% water was initially created. The solution was then mixed in with 2% of acetic acid to put the solution into suspension. 53% of TCP was then added into the suspension and agitated until a homogeneous mixture was reached.

In a third non-limiting example, a solution of 1% of greater than 300 kDa molecular weight chitosan solution (>75% deacetylation) mixed in 43% water was initially created. The solution was then mixed in with 3% of acetic acid to put the solution into suspension. 53% of TCP was then added into the suspension and agitated until a homogeneous mixture was reached.

### Neutral putty formulation - percent by mass (parts/100 parts)

In a non-limiting example, a solution of 1% of greater than 300 kDa molecular weight chitosan solution (>75% deacetylation) mixed in 45% water was initially created. The solution was then mixed in with 1% of acetic acid to put the solution into suspension. The suspension was then neutralized with 3% 2 molar NaOH solution and agitated. 53% of TCP was then added into the suspension and agitated until a putty-like consistency was reached.

### Putty formulation with protein - percent by mass (parts/100 parts)

In a non-limiting example, a solution of 1% of greater than 300 kDa molecular weight chitosan solution (>75% deacetylation) mixed in 45% water was initially created. The solution was then mixed in with 1% of acetic acid to put the solution into suspension. The suspension was then neutralized with 3% 2 molar NaOH solution and agitated. 53% of TCP was then added into the suspension and agitated until a putty-like consistency was reached. The putty was then fully saturated with a protein solution.

### Putty formulation with cells - percent by mass (parts/100 parts)

In a non-limiting example, a solution of 1% of greater than 300 kDa molecular weight chitosan solution (>75% deacetylation) mixed in 45% water was initially created. The solution was then mixed in with 1% of acetic acid to put the solution into suspension. The suspension was then neutralized with 3% 2 molar NaOH solution and agitated. 53% of TCP was then added into the suspension and agitated until a putty-like consistency was reached. The putty was then fully saturated with a physiological fluid containing viable cells.

### Granular powder formulation - percent by mass (parts/100 parts)

In a non-limiting example, a solution of 2% of greater than 300 kDa molecular weight chitosan solution (>75% deacetylation) mixed in 45% water was initially created. The solution was then mixed in with 2% of acetic acid to put the solution into suspension. 51% of TCP was then added into the suspension and agitated until a putty-like consistency was reached. The putty was lyophilized and ground into a powder. The powder was mixed with autograft bone or a physiological fluid intraoperatively to create a gel or putty. The granular powder may be maintained as a powder for later reconstitution.

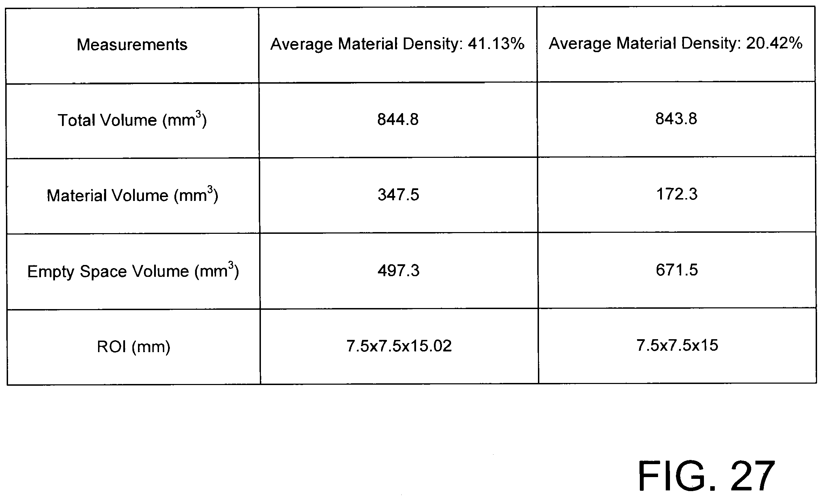

The chitosan/TCP scaffolds exhibited a porosity ranging from about 20 to about 80µm. FIG. 27 provides examples of material properties of 41.13% and 20.42% material density scaffolds including volume of, material volume, empty space volume, and ROI.

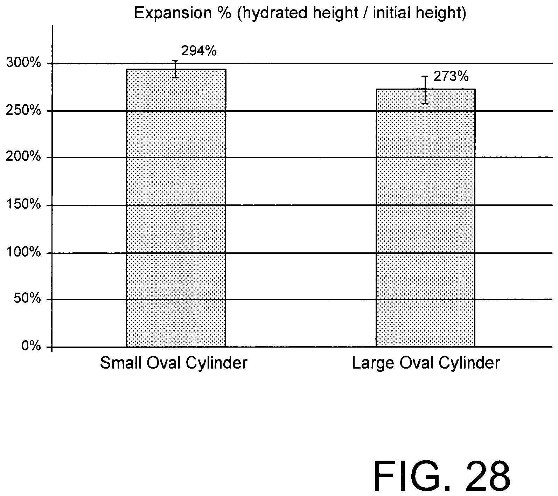

Referring next to FIG. 28, shown is a graph for circumferential expansion in accordance with an exemplary embodiment of a scaffold. In this embodiment, the hydrated dimension was compared to the compressed dimension of the scaffold and the total expansion percentage was calculated based on a 30mg/mL chitosan with 60 mg/mL TCP formulation

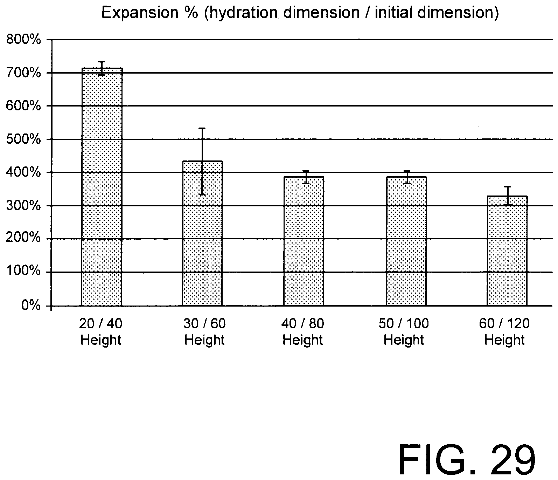

Referring next to FIG. 29, shown is a graph for uniaxial expansion in accordance with an exemplary embodiment of a scaffold. In this embodiment, the hydrated dimension was compared to the compressed dimension of the scaffold. Total expansion percentages were calculated for different formulations including chitosan concentrations of 20, 30, 40, 50, and 60mg/mL corresponding to tri-calcium phosphate concentrations of 40, 60, 80, 100, and 120mg/mL, respectively.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also include individual concentrations (e.g., 1%, 2%, 3%, and 4%) and the sub-ranges (e.g., 0.5%, 1.1%, 2.2%, 3.3%, and 4.4%) within the indicated range. In an embodiment, the term "about" can include traditional rounding according to significant figures of the numerical value. In addition, the phrase "about 'x' to 'y'" includes "about 'x' to about 'y'".

Although the flowcharts depicted in the included drawings show a specific order of execution of the various steps, it is understood that the order of execution may differ from that which is depicted. For example, the order of execution of two or more blocks may be scrambled relative to the order shown. Also, two or more blocks shown in succession may be executed concurrently or with partial concurrence. It should be emphasized that the above-described embodiments of the present disclosure are merely possible examples of implementations set forth for a clear understanding of the principles of the disclosure. Many variations and modifications may be made to the above-described embodiment(s) without departing substantially from the spirit and principles of the disclosure. All such modifications and variations are intended to be included herein within the scope of this disclosure and protected by the following claims.

The polysaccharide may be chitosan.

The chitosan concentration may be greater than about 5%.

The chitosan concentration may be greater than about 30%.

The osteostimulative agent may comprise a calcium salt.

The calcium salt concentration may be greater than about 10%.

The calcium salt concentration may be greater than about 30%.

The calcium salt may be calcium phosphate.

The osteostimulative agent may include granules larger than about 100µm.

The osteostimulative agent may comprise tissue.

The tissue may be allograft, xenograft, or autograft tissue.

The tissue may be at least partially demineralized bone.

The implant may be crosslinked.

The pore size of the implant may be based at least in part upon a control rate freezing.

The pore direction of the implant may be adjusted based at least in part upon directional freezing of a designated surface of the implant.

The implant may be at least partially dehydrated and compressed.

Hydration of the compressed implant may restore the implant to an uncompressed shape.

A malleable orthopaedic implant, comprises: an antimicrobial polysaccharide; an osteostimulative agent; and water.

The polysaccharide may be chitosan.

The chitosan concentration may be less than about 10%.

The chitosan concentration may be less than about 2.5%.

The osteostimulative agent may comprise a calcium salt.

The calcium salt concentration may be greater than about 10%.

The calcium salt concentration may be greater than about 40%.

The osteostimulative agent may comprise tissue.

The tissue may be allograft tissue.

The tissue may be at least partially demineralized bone.

The osteostimulative agent may include granules larger than about 100µm.

The granules may be osteoconductive.

The implant may exhibit anti-microbial activity against staphylococcus aureus (MSRA).

An orthopaedic implant coating comprises: an antimicrobial polysaccharide.

An implant comprises an antimicrobial polysaccharide

A orthopaedic implant comprises: an antibacterial polysaccharide; where the orthopaedic implant is in granular form.

The implant may be configured to form a putty when mixed with autograft bone.

The implant may be configured to form a putty when mixed with a physiological fluid.

The implant may further include an osteostimulative agent.

The osteostimulative agent may comprise a calcium salt.

## Claims

1. A orthopaedic implant scaffold, comprising:
a chitosan solution having a pH level less than about 7, wherein the concentration of chitsan is less than 2.5%; wherein the chitosan comprises about 50% to about 99% deacetylated chitosan; and
and an osteostimulative agent, wherein the osteostimulative agent is a calcium salt, a tissue, or a growth factor.

2. The orthopaedic implant scaffold of claim 1, wherein the implant exhibits anti-microbial activity against staphylococcus aureus.

3. The orthopaedic implant scaffold of claim 1, wherein the growth factor is bound to the scaffold.

4. The orthopaedic implant scaffold of claim 1, wherein the tissue contains a bone morphogenetic protein bound thereto.

5. The orthopaedic implant scaffold of claim 1, wherein the calcium salt concentration is greater than about 10%.

6. The orthopaedic implant scaffold of claim 5, wherein the calcium salt concentration is greater than about 30%.

7. The orthopaedic implant scaffold of claim 5, wherein the calcium salt is calcium phosphate.

8. The orthopaedic implant scaffold of claim 1, wherein the osteostimulative agent includes granules larger than about 100 µm.

9. The orthopaedic implant scaffold of claim 1, wherein the tissue is allograft, xenograft, or autograft tissue.

10. The orthopaedic implant scaffold of claim 9, wherein the tissue is at least partially demineralized bone.

11. The orthopaedic implant scaffold of claim 1, wherein the implant is crosslinked.

12. The orthopaedic implant scaffold of claim 1, wherein the implant is porous and wherein the pore size of the orthopaedic implant scaffold is based at least in part upon a control rate freezing, and pore direction of the orthopaedic implant scaffold is adjusted based at least in part upon direction freezing of a designated surface of the orthopaedic implant scaffold.

13. The orthopaedic implant scaffold of claim 1, wherein the orthopaedic implant scaffold is compressible and is at least partially dehydrated and compressed.

14. The orthopaedic implant scaffold of claim 13, wherein hydration of the compressed orthopaedic implant scaffold restores the implant to an uncompressed state.
